# EUROPÄISCHE PATENTSCHRIFT

(11) **EP 4 367 085 B1**
(45) Veröffentlichungstag und Bekanntmachung des Hinweises auf die Patenterteilung: **19.02.2025**
(21) Anmeldenummer: 22744723.2
(22) Anmeldetag: 06.07.2022
(51) Int. Cl.: C07C 5/48, C07C 11/04, C07C 51/215, C07C 53/08, C07C 67/05, C07C 69/15

(54) **VERFAHREN UND ANLAGE ZUR HERSTELLUNG VON VINYLACETAT**
METHOD AND SYSTEM FOR PRODUCING VINYL ACETATE
PROCÉDÉ ET INSTALLATION DE FABRICATION D'ACÉTATE DE VINYLE

(30) Priorität: 06.07.2021 EP 21020352
(43) Veröffentlichungstag der Anmeldung: 15.05.2024
(73) Patentinhaber: Linde GmbH, 82049 Pullach (DE)
(72) Erfinder: DE VAL, Ricardo Bermejo, 80807 München (DE); SCHUBERT, Martin, 81375 München (DE); MEISWINKEL, Andreas, 83253 Rimsting (DE); HAIDEGGER, Ernst, 85521 Riemerling (DE)
(86) Internationale Anmeldenummer: PCT/EP2022/068799
(87) Internationale Veröffentlichungsnummer: WO 2023/280947

(56) Entgegenhaltungen:
- WO-A1-2018/114747
- WO-A1-2019/175732

## Beschreibung

Die vorliegende Erfindung betrifft ein Verfahren und eine Anlage zur Herstellung von Vinylacetat gemäß den Oberbegriffen der entsprechenden unabhängigen Patentansprüche.

### Hintergrund der Erfindung

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den jeweiligen Olefinen und zu Wasser umgesetzt. Die vorliegende Erfindung betrifft insbesondere die oxidative Dehydrierung von Ethan zu Ethylen, nachfolgend auch als ODHE bezeichnet.

Die ODH(E) kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen und der praktisch irreversiblen Wasserbildung keine thermodynamische Gleichgewichtslimitierung. Die ODH(E) kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine in-situ-Regenerierung ermöglicht bzw. bewirkt. Schließlich werden im Gegensatz zum Steamcracken geringere Mengen an wertlosen Nebenprodukten wie Koks gebildet.

Zu weiteren Details bezüglich der ODH(E) sei auf einschlägige Fachliteratur, beispielsweise Ivars, F. und Löpez Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, sowie X. Li, E. Iglesia, Kinetics and Mechanism of Ethane Oxidation to Acetic Acid on Catalysts Based on Mo-V-Nb Oxides, J. Phys. Chem. C, 2008, 112, 15001-15008, verwiesen.

Insbesondere haben sich für die ODH(E) MoVNb-basierte Katalysatorsysteme als vielversprechend herausgestellt, wie beispielsweise bei F. Cavani et al., "Oxidative dehydrogenation of ethane and propane: How far from commercial implementation?", Catal. Today, 2007, 127, 113-131, erwähnt. Auch zusätzlich Te enthaltende Katalysatorsysteme können verwendet werden. Ist hier von einem "MoVNb-basierten Katalysatorsystem" oder einem "MoVTeNb-basierten Katalysatorsystem" die Rede, sei hierunter ein Katalysatorsystem verstanden, das die genannten Elemente als Mischoxid aufweist, auch ausgedrückt mit MoVNbOₓ bzw. MoVTeNbOₓ. Die Angabe von Te in Klammern steht für dessen optionale Anwesenheit. Die Erfindung kommt insbesondere mit solchen Katalysatorsystemen zum Einsatz.

Bei der ODH werden insbesondere bei Verwendung von MoVNb(Te)Oₓ-basierten Katalysatoren unter industriell relevanten Reaktionsbedingungen signifikante Mengen der jeweiligen Carbonsäuren der eingesetzten Paraffine, im Falle der ODHE insbesondere Essigsäure, als Nebenprodukte gebildet. Für einen wirtschaftlichen Anlagenbetrieb ist daher eine Koppelproduktion von Olefinen und der Carbonsäuren bei Verwendung des beschriebenen Katalysatortyps in der Regel unvermeidlich, eine bevorzugte Bildung von Olefinen ist dabei in der technischen Anwendung üblicherweise wünschenswert.

Abhängig von den Reaktionsbedingungen und verwendeten Katalysatoren entstehen in der ODH(E) neben den bereits erwähnten Nebenprodukten (wobei beispielsweise Essigsäure auch ein weiteres Wertprodukt darstellen kann) ferner gewisse Mengen an Kohlenmonoxid und Kohlendioxid als unerwünschte Nebenprodukte. Derzeitige Verbesserungs- und Optimierungsansätze sind teilweise darauf ausgerichtet, den Anteil an diesen Kohlenstoffoxiden zu minimieren. Aktuell kann dabei der Anteil an Kohlenstoffoxiden auf bis zu weniger als 5% zurückgedrängt werden.

Neuerdings sind auf Grundlage der früheren, eher theoretischen Ansätze bzw. Arbeiten im Labormaßstab kommerzielle Konzepte bekannt geworden, die eben insbesondere auf den genannten Mischoxid-Katalysatoren beruhen. Beispielsweise kann hierbei auf EP 3 519 377 B1 (WO 2018/115416 A1), WO 2018/115418 A1, WO 2018/082945 A1, US 10,730,810 B2 (WO 2018/115494 A1), EP 3 700 663 B1 (WO 2019/081682 A1), WO 2019/243480 A1, US 10,730,811 B2 (WO 2018/115414 A1), WO 2020/187572 A1 der Anmelderin und WO 2018/153831A1, WO 2018/019761 A1 WO 2018/019760 A1, WO 2017/198762 A1, WO 2017/144584 A1, WO 2020/074750 A1, US 9,963,412 B2, US 10,017,432 B2 weiterer Anmelder verwiesen werden. Diesen Konzepten ist gemein, dass dort immer Luft bzw. Sauerstoff als Oxidationsmittel verwendet wird und nur untergeordnete Mengen an Kohlenstoffoxiden im Produktstrom enthalten sind.

Im Hinblick auf nachfolgende Prozesschritte wird bei der ODHE üblicherweise eine hohe Reinheit des Ethylenproduktes (z.B. sog. "Polymer Grade", insbesondere mehr als 99,9%) angestrebt. Daher sind insbesondere ggf. der eigentlichen ODHE nachgeschaltete geeignete Reinigungsschritte und Entfernung von Spurenkomponenten, insbesondere Restgehalte von Sauerstoff aus der ODHE sowie von Kohlenmonoxid und gebildeten Acetylenverbindungen zu berücksichtigen.

Zur Entfernung von Acetylen aus einem Prozessgas von Ethylenanlagen (Steamcrackern) werden z.B. allgemein isotherme Rohgashydrierungen (nach Trocknung, vor einer bekannten C2/C3-Abtrennung, d.h. Deethanisierung), isotherme C2-Hydrierungen (nach C2/C3-Abtrennung, vor C1/C2-Trennung, d.h. Demethanisierung) oder adiabate Tail-End-Hydrierungen (üblicherweise nach C1/C2-Trennung und vor oder nach C2-Splitter) verwendet. Zu Dampfspaltverfahren und bei diesen bekannten Aufbereitungs- und Trennschritten sei beispielsweise auf Fachliteratur wie den Artikel "Ethylene" in Ullmann's Encyclopedia of Industrial Chemistry, Onlineausgabe, 2009, DOI 10.2002/14356007.a10_045.pub3, verwiesen.

Bei der etablierten Steamcrackertechnologie finden die genannten Hydrierungen allesamt in Abwesenheit von molekularem Sauerstoff und bei Kohlenmonoxidgehalten von deutlich weniger als 1% statt (typischerweise weniger als 2.500 ppm, insbesondere weniger als 1.500 ppm). Während hierfür ursprünglich insbesondere Ni-basierte Katalysatoren zum Einsatz kamen, finden für solche Konzepte heutzutage Edelmetallkatalysatoren (insbesondere Pd-basiert) Verwendung, die zusätzlich mit weiteren Metallen, wie Ag, Ce u.a., dotiert sein können. Ebenfalls wird insbesondere in der wissenschaftlichen Literatur über die Verwendung von Rh, Ir, Ru, Pt und Aubasierten Systemen berichtet.

Die Anwesenheit (vergleichsweise) hoher Kohlenmonoxidgehalte, wie bei der ODH(E) der Fall, erfordert daher zumeist andere Ansätze. So ist eine kombinierte Entfernung insbesondere von Kohlenmonoxid und Sauerstoff direkt am Austritt eines ODHE-Reaktors z.B. in US 2010/0256432 A1 und WO 2017/144584 A1 beschrieben worden.

EP 3 708 557 A1 und EP 3 708 558 A1 beschreiben ein Verfahren und geeignete Katalysatoren, die eine zumindest teilweise Entfernung von Acetylen und/oder Sauerstoff aus dem Produktgas einer ODHE ermöglichen. Dabei kommen vorteilhafterweise insbesondere Kupferoxid oder Ruthenium enthaltende Katalysatoren zum Einsatz. Dieser Acetylen- und/oder Sauerstoffentfernung kann eine Kohlendioxidentfernung nachgeschaltet sein. Ein entsprechendes Verfahren kann umfassen, dass ein entsprechendes Prozessgas oder ein Gasgemisch, das unter Verwendung zumindest eines Teils des Prozessgases gebildet wird, in der hier angegebenen Reihenfolge teilweise oder vollständig einer Kondensatabscheidung, einer Verdichtung, einer zumindest teilweisen Entfernung von Sauerstoff und eines oder mehrerer der Acetylene sowie einer oder mehreren Stufen einer Kohlendioxidentfernung unterworfen wird, wobei die zumindest teilweise Entfernung des Sauerstoffs und des oder der Acetylene gleichzeitig und durch eine katalytische Umsetzung unter Einsatz eines Kupferoxid oder Ruthenium enthaltenden Katalysators sowie zumindest in Form einer Hydrierung erfolgt.

Gemäß WO 2018/153831 A1 werden Sauerstoff, Kohlenmonoxid und optional Acetylen aus dem Produktstrom einer ODHE entfernt. Hierbei ist allgemein die Verwendung eines Oxidationskatalysators offenbart, der als Bestandteile insbesondere die Metalle Ni, Cu, Zn, Pd, Ag, Pt, Au, Fe, Mn, Ce, Sn, Ru und Cr aufweisen kann. Dabei werden bevorzugt Cu und/oder Pt-basierte Systeme, insbesondere aber Cu-basierte Systeme, verwendet. In der bereits zuvor zitierten WO 2017/144584A1 beschreibt die gleiche Anmelderin ein Konzept zur Sauerstoffentfernung als ein nachgeschaltetes Bett im ODH-Hauptreaktor, d.h. vor der Prozesskondensatabtrennung, wobei dieses nachgeschaltete Bett mit einem separaten Kühlmittelstrom beheizt/gekühlt wird. Der Katalysator dieses nachgeschalteten Betts kann dabei einerseits die gleiche Zusammensetzung wie der ODH-Katalysator aufweisen oder durch andere Elemente wie Sb, aber bevorzugt Cu, modifiziert sein.

Neben der Anforderung an die hohe Produktreinheit des Ethylenproduktes machen auch nachgeschaltete kryogene Anlagenteile eine quantitative Abscheidung von Kohlendioxid notwendig. Kohlendioxid kann - insbesondere in Gehalten, wie sie bei der ODHE auftreten - aufgrund seiner hohen Wechselwirkung mit geeigneten Lösungsmitteln bzw. Waschflüssigkeiten ebenso vergleichsweise einfach aus dem Produktgemisch entfernt werden, wobei bekannte Verfahren zur Kohlendioxidentfernung, insbesondere entsprechende Wäschen (beispielsweise Aminwäschen) zum Einsatz kommen können. Die beladene Waschflüssigkeit wird dann in einer separaten Kolonne regeneriert und dabei wird sehr reines Kohlendioxid durch Desorption freigesetzt.

Sollten Folgeschritte die Abwesenheit oder nur eine sehr geringe Restkonzentration von Kohlendioxid erfordern (z.B. aufgrund einer Katalysatorinhibierung oder - vergiftung), kann der Restgehalt an Kohlendioxid nach einer Aminwäsche durch eine optionale Laugewäsche als Feinreinigung anforderungsgemäß weiter reduziert werden.

Mit gewissen Ausnahmen können die entsprechenden Waschflüssigkeiten auch mit Sauerstoff reagieren, wodurch es im Laufe der Zeit zu einer nachteiligen Alterung bzw. Schädigung der Waschmittel kommen kann, die einen kontinuierlichen Purge- und Makeup-Strom erfordern bzw. zu einer unerwünschten Verkürzung der Lebensdauer dieser Waschflüssigkeiten führen. Daher ist aus diesem Aspekt die Entfernung von Sauerstoff stromauf einer entsprechenden Wäsche vorteilhaft.

Die Entfernung von Wasser erfolgt gemäß Stand der Technik typischerweise mittels regenerativer Trockner auf Molsiebbasis und ist neben der Erreichung der Produktspezifikation ebenfalls zwingend in Hinblick auf nachfolgende kryogene Prozesschritte, um dort Verlegungen durch Abscheidung von Eis zu vermeiden.

Typischerweise muss im Rahmen einer geeigneten Verfahrensführung bei einem ODHE-Prozess auch im Produktstrom enthaltenes Methan (z.B. insbesondere aus dem Ethaneinsatzstrom der ODHE stammend) entfernt werden. Dazu kommt üblicherweise eine C1/C2-Trennung ("Demethanizer" oder Demethanisierung) an sich bekannter Art zum Einsatz, die entsprechende kryogene Bedingungen erfordert. Ein solcher Demethanizer entfernt dabei gleichzeitig Kohlenmonoxid aus dem Produktstrom. Es wird also eine C1minus-Fraktion gebildet, die als wesentliche Bestandteile Methan und/oder Kohlenmonoxid enthält. Allgemein soll hier unter einer "C1minus-Fraktion" eine Fraktion verstanden werden, die überwiegend oder ausschließlich Methan und tiefer als Methan siedende Komponenten aufweist, wie z.B. insbesondere Kohlenmonoxid und Sauerstoff. Entsprechend handelt es sich bei einer "C2plus-Fraktion" um eine Fraktion, die überwiegend oder ausschließlich Komponenten mit höheren Siedepunkten als Methan aufweist. Die Angabe "überwiegend oder ausschließlich" oder "reich" soll dabei einen Anteil von wenigstens 75%, 80%, 85%, 90%, 95% oder 99% auf Volumen-, Masse- oder Molbasis bezeichnen. Entsprechende Zahlenwerte gelten auch dann, wenn von einem "überwiegenden Teil" die Rede ist, beispielsweise in Bezug auf beliebige Produkt- oder Folgeströme. Ein "überwiegender Teil" bezeichnet insbesondere mehr als 50%, mehr als 66%, mehr als 75% oder mehr als 90% (bezogen auf ein Teilvolumen an einem Gesamtvolumen oder einer Teilmenge an einer Gesamtmenge und dergleichen). Wird eine Fraktion mit dem Namen der enthaltenen Hauptkomponente ("Ethanfraktion", "Ethylenfraktion" und dergleichen bezeichnen, muss diese nicht notwendigerweise ausschließlich aus dieser bestehen, sondern kann auch im erläuterten Sinne diese Komponente überwiegend aufweisen oder reich an dieser sein.

Im Eintrittsstrom des Demethanizers noch enthaltene Spuren von Sauerstoff gelangen dabei ebenfalls in diese C1minus-Fraktion. Daher wird üblicherweise angestrebt, den Eintrittsgehalt an Sauerstoff im Eintrittsstrom des Demethanizers zu begrenzen und so die mögliche Bildung einer explosionsfähigen Atmosphäre am Kopf einer hier verwendeten Kolonnezu vermeiden (vgl. hierzu auch WO 2018/082945 A1). EP 3 456 703 A1 beschreibt beispielsweise einen Demethanizer im Zerlegungsteil einer ODHE-Anlage, der (auch) zu diesem Zweck mit einer Druckwechseladsorption im Kopfstrom kombiniert wird.

Schlussendlich muss nicht umgesetztes Ethan von Ethylen getrennt werden, was mittels eines C2-Splitters, der ebenfalls unter kryogenen Bedingungen betrieben wird, erfolgt. Dieser muss also so gebaut und betrieben werden, dass Ethan im Ethylen praktisch quantitativ entfernt wird und gleichzeitig ein Ethanstrom, der zur ODHE zurückgeführt wird, möglichst wenig bzw. kein Ethylen enthält.

Eine Hauptanwendung für Vinylacetat (VAM) ist die Polymerisation zu Polyvinylacetat (PVAc) oder zu anderen Copolymerisaten. Vinylacetat stellt somit ein wichtiges Intermediat der chemischen Industrie dar.

Das heutzutage übliche Herstellungsverfahren geht dabei von Ethylen und Essigsäure aus und wird z.B. in dem Artikel "Vinyl Esters" in Ullmann's Encyclopedia of Industrial Chemistry, Onlineausgabe, 2019, DOI 10.1002/14356007.a27_419.pub2, beschrieben. Diese werden in einer exothermen Reaktion unter Sauerstoffzusatz in einem Gasphasenprozess umgesetzt. Dabei werden üblicherweise Festbettreaktoren mit Pd-Katalysatoren verwendet. Als Nebenreaktion ist insbesondere die oxidative Umsetzung von Ethylen zu Kohlendioxid zu erwähnen. Ethylen und Essigsäure werden bevorzugt in einem Verhältnis von 2 bis 3 zu 1 eingesetzt und es wird Sauerstoff im Verhältnis von bis zu 0,5 Äquivalenten in Bezug auf Essigsäure zugegeben. Typische Reaktionsbedingungen betragen 120 °C bis 180 °C bei einem Druck von 5 bar bis 12 bar. Typischerweise wird dabei ein Inertgas wie beispielsweise Kohlendioxid (beispielsweise 10 bis 30%), Stickstoff oder Argon zugesetzt, um die Reaktionswärme und Zündbereiche besser zu beherrschen. Grundsätzlich ist aber auch eine Synthese von Vinylacetat ohne Zusatz von Kohlendioxid oder mit deutlich niedrigerem Anteil an Kohlendioxid als zuvor genannt möglich.

Verunreinigungen im Feedstrom der Vinylacetatsynthese, insbesondere Acetylen und Kohlenmonoxid, haben dabei eine negative Auswirkung auf die Vinylacetatsynthese. Der Feed einer Vinylacetatsynthese ist also idealerweise frei von diesen Verbindungen bzw. enthält diese nur in äußerst geringen Konzentrationen. Ein geeigneter Ethylenfeed für die Vinylacetatsynthese kann nach dem Stand der Technik daher insbesondere als hochaufgereinigtes Ethylen (z.B. "Polymer Grade") aus entsprechenden Verfahren wie beispielsweise dem Steamcracking und einem nachgeschalteten Aufreinigungs- und Zerlegungsteil bereitgestellt werden.

Der eigentlichen Synthese von Vinylacetat ist sodann eine Aufbereitung nachgeschaltet, die insbesondere eine Phasentrennung umfasst (Trennung Gasphase und flüssiges Rohprodukt) sowie vorteilhafterweise auch eine Wäsche, die insbesondere zur Effizienzsteigerung in der Ausbeute von Vinylacetat mittels Extraktion mit Essigsäure dient, die aber auch Grundlage einer Rückgewinnung von Ethylen und ggf. Kohlendioxid als Einsatzstoffen für die Synthese des Vinylacetats ist. Zumeist wird in diesem Strom oder einem Teilstrom dann eine separate absorptive Kohlendioxidentfernung vorgenommen, um den Kohlendioxidgehalt einstellen zu können. In dem erwähnten Artikel in Ullmann's Encyclopedia of Industrial Chemistry wird hierzu insbesondere eine regenerative Kaliumcarbonatlösung aufgeführt. Für die nachfolgende Aufreinigung des Vinylacetat-Rohproduktes kommen verschiedene destillative Lösungen zum Einsatz, die detailliert in dem genannten Artikel dargelegt werden. Die Aufbereitung (Phasentrennung, Wäsche, absorptive Entfernung von Kohlendioxid und Destillation) erfolgen dabei üblicherweise bei einem niedrigeren Druck als die Synthese des Vinylacetats.

Prinzipiell sind Ansätze zu einer verfahrenstechnischen Integration von ODHE und Vinylacetatsynthese bekannt. Diese erscheinen grundsätzlich vorteilhaft, da die ODHE üblicherweise Essigsäure als Koppelprodukt liefert und die Vinylacetatsynthese wiederum von Ethylen und Essigsäure ausgeht.

Die vorliegende Erfindung stellt sich die Aufgabe, verbesserte und effektivere Möglichkeiten zur verfahrenstechnischen Integration von ODHE und Vinylacetatsynthese aufzuzeigen.

### Offenbarung der Erfindung

Die vorstehend genannte Aufgabe wird durch die in den jeweiligen unabhängigen Patentansprüchen angegeben Maßnahmen gelöst. Bevorzugte Ausgestaltungen sind jeweils Gegenstand der abhängigen Patentansprüche und der nachfolgenden Beschreibung.

Nachfolgend sollen zunächst bekannte Verfahren, die eine verfahrenstechnische Integration von Vinylacetatsynthese und ODHE umfassen, erläutert werden, um diesen gegenüber Vorteile und Merkmale der vorliegenden Erfindung aufzuzeigen.

Werden nachfolgend Druckwerte in der Einheit bar angegeben, handelt es sich, falls nicht anders erwähnt, um Absolutdrücke. Druckunterschiede sind in bar ausgedrückt.

WO 2018/114747 A1 offenbart unter anderem eine Entfernung von Kohlendioxid und einen der Vinylacetatsynthese und Kohlendioxidentfernung nachgeschalteten C2-Splitter mit Rückführungen von Ethylen in die Vinylacetatsynthese und von Ethan in die ODHE. Es wird aber immer mindestens ein Teilstrom aus der ODHE - der also verfahrensbedingt auch Kohlendioxid enthält - in die Vinylacetatsynthese eingebracht. Lediglich ein Bypass der Vinylacetatsynthese ermöglicht die direkte Überführung eines Teilstromes aus der ODHE in die Kohlendioxidentfernung. Eine solche Verschaltung bedeutet, dass nur eine eingeschränkte Variationsmöglichkeit des Kohlendioxidgehaltes möglich ist. Insbesondere bei Veränderungen im Umsatz- und Produktverteilungsverhalten der ODHE kann es dabei zu unerwünschten Veränderungen kommen. Insbesondere ist somit auch keine vollständige Abtrennung des Kohlendioxids im Eintrittsstrom der Vinylacetatsynthese und/oder Anreicherung auf sehr geringe Restgehalte an Kohlendioxid möglich. Zudem finden sich keine Angaben zur vorteilhaften Ausführung der jeweiligen Druckniveaus der einzelnen Reaktions- und Trennschritte sowie einer entsprechenden notwendigen Verdichtung der Prozess- und insbesondere Rückführströme. Insbesondere legt diese Schrift ein abfallendes Druckgefälle nahe, wobei die Kohlendioxidentfernung also bei niedrigerem Druck als die Vinylacetatsynthese erfolgt.

WO 2018/114752 A1 offenbart ebenfalls eine Integration von ODHE und Vinylacetatsynthese. Schwerpunkt ist dabei die Nutzung einer Dampfpermeation zur Abtrennung von Wasser aus dem Austrittsstrom der ODHE. Ausführungsformen der weiteren Prozessführung sind ansonsten stark an die zuvor genannte Schrift angelehnt und offenbaren auch hier keine weiteren Lösungen im Sinne der vorliegenden Erfindung. Keine der beiden vorgenannten Schriften geht auf die Anwesenheit von Methan in den Prozesströmen (insbesondere resultierend aus Methan im Feed der ODHE) ein, und es sind somit auch keine Ansätze zur Methanabtrennung offenbart.

Insbesondere gehen diese Schriften auch nicht auf den Einfluss von Spurenkomponenten, insbesondere Kohlenmonoxid und Acetylen, auf die Vinylacetatsysnthese ein. Eine Lösung zur Entfernung dieser Spurenkomponeten wird nicht vorgeschlagen.

In den vorgenannten Schriften ist die WO 1998/005620 A1 zitiert, die eine sequentielle Verschaltung von ODHE und Vinylacetatsynthese beschreibt. Auch diese Schrift fokusiert jedoch insbesondere auf die Abtrennung und ggf. Rückführung von Essigsäure und/oder Vinylacetat bzw. entsprechend angereicherten Teilströmen. Spezifische Ausführungen zu einer Kohlendioxidentfernung offenbart diese Schrift nicht. Üblicherweise wird der Austrittsstrom der ODHE ohne eine Kohlendioxidentfernung der Vinylacetatsynthese zugeführt. Es erfolgt hier also keinerlei Entfernung, Abreicherung oder Anpassung des Kohlendioxidgehaltes im Eintrittsstrom der Vinylacetatsynthese. Auf die Anwesenheit von Methan in den Prozesströmen geht auch diese Schrift nicht ein, und enthält somit auch keinen Ansatz zur Methanabtrennung in einem integrierten Verfahren.

Gemäß einer in WO 1998/005620 A1 offenbarten Variante kann in der ODHE Kohlenmonoxid zu Kohlendioxid oxidiert werden. Hierfür ist jedoch ein Katalysator notwendig, der einen ODHE-Produktstrom mit sehr geringen Anteilen an Kohlenmonoxid entstehen läßt. Wie zuvor dargestellt, erfüllen typische ODHE-Katalysatoren, insbesondere MoVNb-basierte Katalysatorsysteme, diese Anforderung (noch) nicht. Somit offenbart dieser Ansatz keine Lösung zu einer ausreichenden Kohlenmonoxidentfernung im Sinne einer Integration von ODHE und Vinylacetatsynthese. Gemäß einer weiteren Variante wird in der Vinylacetatsynthese ein Pd-haltiger Katalysator eingesetzt wird, der auch Kohlenmonoxid in einer geeigneten Reaktion umsetzt. Wie in der Schrift beschrieben, ist diese Gestaltung jedoch auf Pd-basierte Katalysatoren beschränkt. Auch wenn die heutzutage für die Vinylacetatsynthese verwendeten Katalysatoren typischerweise Pd-basiert sind, ergibt sich durch die zusätzliche Kohlendioxidentfernung und Kopplung der Reaktionsschritte in der Vinylacetatsynthese eine signifikante Einschränkung in den Prozessparametern, z.B. bei Veränderungen in der Zusammensetzung des Eintrittsstromes, da sich das Verhalten der Kohlenmonoxidentfernung und der Vinylacetatsynthese nicht unabhängig voneinander beeinflussen und einstellen lassen.

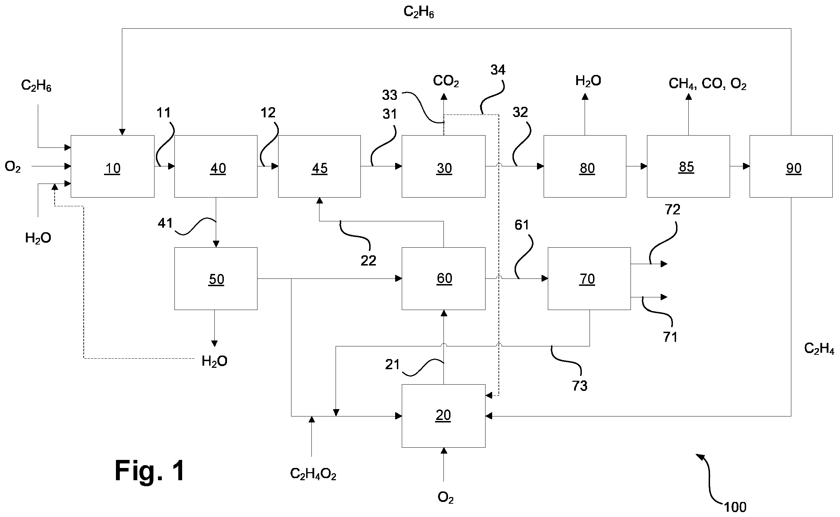

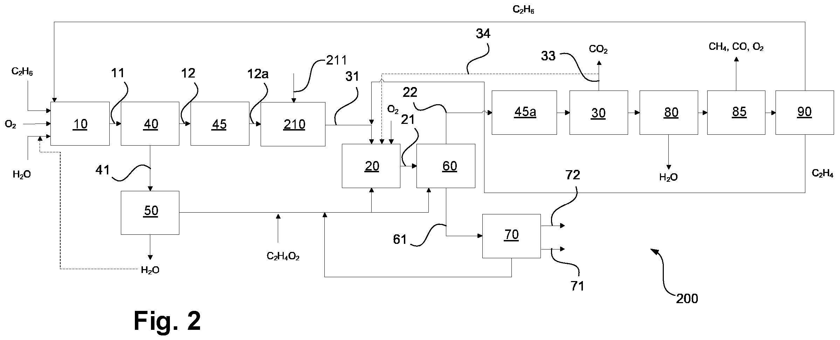

WO 2000/069802 A1 ist ebenfalls in den vorstehend genannten Schriften zitiert und beschreibt die sequentielle Integration einer ODHE und einer Vinylacetatsynthese, insbesondere mit MoVNb-basierten Katalysatoren für die ODHE und insbesondere Pdbasierten Katalysatoren für die Vinylacetatsynthese. In dieser Schrift wird eine Kohlendioxidentfernung entweder nur in dem Gasstrom stromab der ODHE, also stromauf der Vinylacetatsynthese, vorgesehen (dortige Figur 1) oder aber das im Gasstrom nach der ODHE enthaltene Kohlendioxid wird vollständig in die Vinylacetatsynthese überführt und eine Kohlendioxidentfernung erfolgt erst stromab der Vinylacetatsynthese (Figur 2). Auf die Anwesenheit von Methan in den Prozessströmen geht auch diese Schrift nicht ein und enthält somit auch keinen Ansatz zur Methanabtrennung in einem integrierten Verfahren. Diese Schrift offenbart keine Lösungen zu einer Spurenentfernung von z.B. Kohlenmonoxid, Acetylen und/oder Sauerstoff. Ein entsprechender Zwischenschritt wird anspruchsgemäß explizit ausgeschlossen. Es wird daher auch keine geeignete technische Lösung für die Aufgabe einer Kohlenmonoxidentfernung offenbart. Vielmehr zielt die Schrift auf eine Ausführungsform der ODHE ab, bei der kein Kohlenmonoxid entsteht, was, wie zuvor ausgeführt, auch heutzutage nicht dem Stand der Technik entspricht.

WO 2001/090042 A1 beschreibt ebenfalls eine Integration einer ODHE und einer Vinylacetatsynthese. Dabei kommen für die ODHE insbesondere Pd-dotierte Katalysatoren vom Typ MoₐPd_{b}X_{c}Y_{d} zum Einsatz. Eine Kohlendioxidentfernung gemeinsam mit "Inerten" lediglich aus einem Recyclestrom der Vinylacetatsynthese zur ODHE wird nur ohne weitere Ausführungen in einer Skizze angedeutet. In einer schematischen Zeichnung wird eine der Vinylacetatsynthese vorgeschaltete Kohlenmonoxidentfernung skizziert, ohne dass jedoch vorteilhafte Lösungsansätze hierfür aufgezeigt werden. Gemäß Beschreibung entsteht in der ODHE kein Kohlenmonoxid oder nur eine Menge von weniger als 100 ppm, was nicht dem Verhalten unter technisch relevanten Bedingungen (vgl. oben) entspricht. Ausführungen hinsichtlich Acetylen im Produktstrom der ODHE und einer Entfernung stromauf der Vinylacetatsynthese finden sich nicht.

Weitere Druckschriften, die ebenfalls entsprechende Kombinationen, nicht aber die erfindungsgemäß vorgeschlagenen Maßnahmen offenbaren oder andeuten, sind z.B. US 2005/0148791 A1, US 5,066,365 A, US 2014/066650 A1 und US 2002/058849 A1.

Unabhängig von den vorgenannten Dokumenten beschreibt auch WO 2019/175732 A1 eine Verknüpfung einer ODHE unter Verwendung verschiedener Katalysatoren (u.a. kommen auch MoVTeNbOx-basierte Systeme zum Einsatz) mit einer Vinylacetatsynthese. Dabei erfolgt eine Oxidation von Kohlenmonoxid und Acetylenentfernung unmittelbar im Gasphasenanteil des Austrittsstromes der ODHE unter Verwendung mindestens eines Metalls oder eines entsprechenden Metalloxids ausgewählt aus den Gruppe 11, 4, 7 oder 9, den Lanthaniden oder Actiniden - explizit benannt werden insbesondere Cu, Ag, Au bzw. Kombinationen davon. Detaillierte Ausführungen erstrecken sich jedoch nur auf Metalle der Gruppe 11 und die optionale Verwendung von CeO₂ oder ZrO₂ als Promoter. Metalle oder deren Oxide aus den Gruppen 8 (insbesondere Ru) und 10 (insbesondere Ni, Pd, Pt) werden hingegen an keiner Stelle der Anmeldeschrift als geeignet für diesen Zweck benannt. Die Anmeldeschrift geht an keiner Stelle auf die vorteilhafte Positionierung - insbesondere in Hinblick auf das Druckniveau - der beanspruchten Oxidation von Kohlenmonoxid ein. Eine weitergehende Integration der beiden Verfahren wird nicht offenbart. Hinsichtlich einer Kohlendioxidentfernung finden sich in dieser Schrift nur recht allgemeine Hinweise auf eine nicht näher spezifizierte "inert removal unit". Diese beinhaltet jedoch keine selektive Kohlendioxidentfernung und ermöglicht auch keine bedarfsgerechte Einstellung eines Anteils einer einzelnen oder aller Inertkomponenten im Feedstrom der Vinylacetatsynthese, insbesondere nicht die bedarfsgerechte Einstellung des Kohlendioxidgehalts. Vielmehr wird eine gemeinsame, d.h. unselektive Entfernung aller Inertkomponenten in einem Schritt vorgeschlagen. Eine solche Entfernung ist, wie dem Fachmann bekannt, gemäß Stand der Technik insbesondere nicht mittels einer absorptiven Wäsche möglich. Gemäß speziellen Ausgestaltungen sind Sauerstoff und Acetylen im Feedstrom der Vinylacetatsynthese dabei praktisch vollständig abwesend.

Die Entfernung von Kohlendioxid erfolgt herkömmlicherweise insbesondere mittels Absorption (z.B. regenerierbare Aminwäschen und nicht regenerierbare Laugewäschen). Kohlendioxid kann dabei aufgrund seiner hohen Wechselwirkung mit geeigneten Lösungsmitteln bzw. Waschflüssigkeiten vergleichsweise einfach aus dem Produktgemisch entfernt werden, wobei auch im Rahmen der Erfindung bekannte Verfahren zur Kohlendioxidentfernung, insbesondere entsprechende Wäschen (beispielsweise Aminwäschen) zum Einsatz kommen können. Das beladene Waschmittel bei Aminwäschen wird dann in einer separaten Kolonne regeneriert, wobei im Wesentlichen reines Kohlendioxid durch Desorption freigesetzt wird und als relativ reiner Strom für eine weitere Verwendung zur Verfügung steht. Eine weitere Feinreinigung und Reduzierung des Kohlendioxidgehaltes ist durch eine Laugewäsche möglich. Kohlendioxid kann daher also bedarfsweise abgetrennt und in der zugehörigen Regenerierung nahezu als Reinsubstanz gewonnen werden. Eine absorptive Kohlendioxidentfernung findet aufgrund der mit dem Druck steigenden Löslichkeit vorzugsweise, und insbesondere auch im Rahmen der vorliegenden Erfindung, bei erhöhtem Druck, also mehr als 5 bar, insbesondere mehr als 10 bar und insbesondere mehr als 15 bar statt.

Im Rahmen einer vorteilhaften Trenn- und Aufreinigungssequenz können im Rahmen der vorliegenden Erfindung insbesondere auch kryogene und nicht-kryogene Destillationsverfahren zum Einsatz kommen, wobei erfindungsgemäß ein Demethanizer und ein C2-Splitter eingesetzt werden.

In einem sogenannten Demethanizer wird eine leichte(re) Fraktion (C1minus-Fraktion) als Kopfstrom gebildet, während eine schwere(re) (C2plus-Fraktion) als Sumpfstrom gewonnen wird. Die C1minus. Fraktion ist dabei insbesondere angereichert an Methan und/oder Kohlenmonoxid, während die Sumpffraktion an diesen Komponenten abgereichert oder frei von diesen Komponenten ist. Der Begriff "angereichert" soll dabei hier jeglichen erhöhten Gehalt, aber insbesondere einen um mindestens den Faktor 2, 5, 10, 100 oder mehr erhöhten Gehalt, an einer oder mehrerer Komponenten, bezogen auf ein Ausgangsgemisch vor der Anreicherung, bezeichnen. Entsprechend soll der Begriff Begriff "abgereichert" hier jeglichen verringerten Gehalt, aber insbesondere einen um mindestens den Faktor 2, 5, 10, 100 oder mehr verringerten Gehalt, an einer oder mehrerer Komponenten, bezogen auf ein Ausgangsgemisch vor der Abreicherung, bezeichnen.

Es sind also im Falle einer ODHE in der Sumpffraktion überwiegend oder ausschließlich Ethan und Ethylen enthalten. Ein Demethanizer wird typischerweise, und insbesondere auch im Rahmen der vorliegenden Erfindung, in einem Druckbereich zwischen 10 bar und 50 bar, bevorzugt 15 bar und 40 bar, besonders bevorzugt zwischen 20 bar und 35 bar, betrieben. Insbesondere die Kopftemperatur muss zur Gewährleistung eines flüssigen Rücklaufs entsprechend tief eingestellt werden. Typische Temperaturen liegen hier unterhalb von -50 °C, insbesondere unterhalb von -70 °C und weiter insbesondere unterhalb von -80 °C.

In EP 3 339 277 A1 finden sich Ausführungen zum Demethanizer eines ODHE-Prozesses. In EP 3 456 703 A1 wird beispielsweise ein ODHE-Verfahren beschrieben, das zunächst einen Demethanizer im Zerlegungsteil umfasst. Der hier gebildete Kopfstrom umfassend insbesondere Methan wird einer Druckwechseladsorption (PSA) zugeführt, um in diesem Strom enthaltenes Ethylen zurückzugewinnen und in den Zerlegungsteil der ODHE-Anlage zurückzuführen.

Ferner ist die Trennung von Ethylen und nicht umgesetztem Ethan in einem sogenannten C2-Splitter erforderlich. Ethylen wird dabei typischerweise in einer Reinheit von mehr als 98%, bevorzugt mehr als 99%, besonders bevorzugt mehr als 99,5%, gewonnen. Ethan wird sowohl in Steamcracking- als auch in ODHE-Verfahren als Reaktionseinsatz zurückgeführt, also z.B. wieder in den ODHE-Reaktor recycliert. Um hier einen Verlust an Ethylen zu minimieren, wird auch diese Fraktion üblicherweise in hoher Reinheit gewonnen. Ein C2-Splitter wird typischerweise, und insbesondere auch im Rahmen der vorliegenden Erfindung, in einem Druckbereich zwischen 5 bar und 50 bar, bevorzugt 7 bar und 40 bar, besonders bevorzugt zwischen 8 bar und 35 bar, betrieben Auch hier ist - in Abhängigkeit vom verwendeten Druck - eine relativ niedrige Kopftemperatur erforderlich, also typischerweise weniger als -5 °C, insbesondere weniger als -20 °C und weiter insbesondere weniger als -50 °C.

Als weitere Trenn- und Aufreinigungsschritte sind (als nicht abschließende Aufzählung) Phasentrennungen, insbesondere mittels Abscheidern und Dekantern, reaktive, insbesondere katalytische Entfernungen von Sauerstoff und Spurenverbindungen (insbesondere Kohlenmonoxid und Acetylen), insbesondere unter Zusatz von Wasserstoff und/oder Sauerstoff, adsorptive Reinigungs- und Trocknungsverfahren, die je nach Material und Verwendungszweck regenerierbare und nicht regenerierbare Adsorbentien nutzen können, und Druckwechseladsorptions- (PSA-) und Membranverfahren bekannt und können auch im Rahmen der vorliegenden Erfindung zum Einsatz kommen.

### Merkmale und Vorteile der Erfindung

Die bereits oben angegebene Aufgabe der vorliegenden Erfindung lässt sich insbesondere in einem ersten Aspekt in der Schaffung einer Prozessführung konkretisieren, die eine besonders effiziente Integration von ODHE und Vinylacetatsynthese und somit eine Nutzung des Produktgemisches der ODHE für die Vinylacetatsynthese ermöglicht.

Der Ausgangspunkt ergibt sich dabei aus dem Stand der Technik wie folgt:
- Eine "konventionelle" ODHE liefert neben Ethylen insbesondere Essigsäure als Koppelprodukt. Das Verhältnis von Ethylen und Essigsäure lässt sich dabei innerhalb gewisser Grenzen bedarfsgerecht einstellen.
- Am Austritt des ODHE-Reaktors ist üblicherweise ein Mindestgehalt an Sauerstoff zu gewährleisten, um eine Katalysatorschädigung zu vermeiden. Dieser Restsauerstoffgehalt wird jedoch gemäß dem Stand der Technik möglichst minimiert und beträgt im gesamten Austrittsstrom der ODHE (also im feuchten Prozessgasstrom vor der Kondensatabtrennung) üblicherweise mindestens 0,41 mol%, maximal jedoch weniger als 1,5 mol%, weniger als 0,8 mol% oder weniger als 0,6 mol%. Nachgelagert können sodann Maßnahmen zu einer Sauerstoffentfernung vorgesehen sein, wie z.B. in den bereits zuvor unter genannten Schriften (z.B. US2010/0256432 A1) beschrieben.
- Üblicherweise wird Ethylen als Reinprodukt bereitgestellt und entsprechend aufwendig von anderen Kohlenwasserstoffen, insbesondere z.B. Ethan und Methan, befreit. Methan kann dabei insbesondere als Bestandteil des Feedstroms der ODHE eingetragen werden.
- Die Vinylacetatsynthese benötigt Ethylen und Essigsäure als Reaktionseinsatz, typischerweise in einem Verhältnis von 3 zu 1 bis 1 zu 1.
- Andererseits muss für die Vinylacetatsynthese Sauerstoff als Reaktand zugesetzt werden.

Für eine weitere Integration der beiden Prozesse gilt es jedoch insbesondere die folgenden Aspekte zu berücksichtigen, für die die vorliegende Erfindung, insbesondere auch in ihren Ausgestaltungen, vorteilhafte Lösungen angibt:
- Neben Ethylen und Essigsäure entstehen in der ODHE auch weitere Nebenprodukte, neben Kohlendioxid insbesondere Acetylen und Kohlenmonoxid, die durch geeignete Verfahrensschritte zu entfernen sind. Insbesondere Acetylen und Kohlenmonoxid haben dabei eine negative Auswirkung auf nachfolgende Verfahrensschritte, so auch hier auf die Vinylacetatsynthese.
- In der Vinylacetatsynthese wird üblicherweise ein geeignetes Verdünnungsmittel zugesetzt, welches im Anschluss wiederum aus dem Produktgemisch der Vinylacetatsynthese entfernt werden muss. Häufig wird für diese Aufgabe Kohlendioxid genutzt und zusätzlich entsteht in der Vinylacetatsynthese weiteres Kohlendioxid als Nebenprodukt. Entsprechend muss auch der Produktstrom aus der Vinylacetatsynthese einer Kohlendioxidreinigung unterworfen werden. Der Eintrittsstrom der Vinylacetatsynthese kann aber auch insbesondere kein oder nur geringe Mengen an Kohlendioxid enthalten.
- Insbesondere eine prozessoptimierte Verfahrensführung von Kohlendioxid ist dabei bislang nicht Bestandteil verfügbarer Technologien oder Entwicklungen. Kohlendioxidwird typischerweise vielmehr durch isolierte Reinigungsschritte entfernt und nicht innerhalb eines integrierten Gesamtprozesses verwertet.
- Insbesondere die Verdichtung von Gasströmen erfordert hohen Aufwand hinsichtlich der erforderlichen aufwendigen Apparate (Kompressoren, oftmals mehrstufig ausgeführt) und Betriebskosten (Energieaufwand für die Verdichtung). Eine sequentielle Verschaltung unabhängiger ODHE- und Vinylacetatsyntheseprozesse macht ein mehrfaches Verdichten und Entspannen über die Prozesskette hinweg erforderlich. Aber auch bekannte Ansätze zur Prozessintegration von ODHE und Vinylacetatsynthese weisen keinen optimierten Ansatz für die Wahl geeigneter Prozessführungen und optimierter Druckniveaus auf.

Wie erwähnt, wird in der ODH als Neben- bzw. Koppelprodukt eine Carbonsäure, im Falle von Ethan als Einsatz in die ODH (also in der ODHE) Essigsäure, gebildet. Eine Einstellung des Verhältnisses Ethylen zu Essigsäure ist durch geeignete Maßnahmen, wie in WO 2018/115418 A1, WO 2018/115416 A1 und WO 2019/243480 A1 beschrieben, wie die Wahl des Katalysators oder der Reaktionsbedingungen, insbesondere der Einstellung des Wasserpartialdrucks im Prozessgasstrom der ODHE (vgl. hier insbesondere WO 2018/115416 A1) möglich.

Im Rahmen der vorliegenden Erfindung wird dabei insbesondere ein Verhältnis von Ethylen zu Essigsäure zwischen 1 zu 1 und 10 zu 1, insbesondere zwischen 2 zu 1 und 5 zu 1 und weiter insbesondere zwischen 2,5 zu 1 und 4 zu 1 angestrebt. Diese Essigsäure kann, zusammen mit Reaktionswasser, vergleichsweise einfach durch eine Kondensation und/oder eine Wasserwäsche aus einem entsprechenden Produktgemisch der oxidativen Dehydrierung abgetrennt werden. Es erfolgt also eine Auftrennung des Produktstromes in eine Kondensatphase und eine Gasphase.

Durch geeignete, dem Fachmann bekannte Verfahren ist bedarfsweise dann auch eine Anreicherung der Essigsäure aus der Kondensatphase und Gewinnung als eigenes Wertprodukt möglich, welches der Vinylacetatsynthese zugeführt werden kann. Die Gasphase aus dem Produktstrom der ODHE enthält sodann insbesondere Ethylen, aber auch weitere Bestandteile wie nicht umgesetztes Ethan, Methan (insbesondere aus dem Feed der ODHE), und insbesondere Kohlenmonoxid und Kohlendioxid sowie geringe Mengen an Acetylen (weniger als ca. 300 bis 400 ppmv) als Nebenprodukte.

Die oxidative Dehydrierung wird im Rahmen der vorliegenden Erfindung vorteilhafterweise auf einem Druckniveau von 1 bar bis 10 bar, insbesondere 2 bar bis 6 bar, und die Vinylactetatherstellung vorteilhafterweise auf einem Druckniveau von 2 bar bis 50 bar, insbesondere 5 bar bis 15 bar, durchgeführt.

Aufbauend auf diese Grundlagen offenbart die vorliegende Erfindung in einem Hauptaspekt eine optimierte Lösung für eine effiziente Kohlendioxidentfernung aus einer integrierten ODHE und Vinylacetatsynthese. Diese ermöglicht insbesondere auf einfache Weise die bedarfsgerechte Einstellung des Kohlendioxidanteils im Eintrittsstrom der Vinylacetatsynthese. Die Erfindung macht sich dabei in geschickter Weise die folgenden Tatsachen zu Nutze:
1. In der Vinylacetatsynthese kann ein gewisser Anteil von Kohlendioxid im Reaktionseinsatz der Vinylacetatsynthese als Verdünnungsmittel verwendet werden, Kohlendioxid kann also die Vinylacetatsynthese durchlaufen bzw. ist hier ggf. in bestimmten Anteilen sogar erwünscht. Der Kohlendioxid-Anteil im Feed zur Vinylacetatsynthese kann dabei 0 bis 30%, insbesondere 0 bis 15%, weiter insbesondere 0 bis 5% betragen.
2. In der Vinylacetatsynthese entsteht ohnehin (weiteres) Kohlendioxid als Nebenprodukt.
3. Kohlendioxid kann aufgrund seiner hohen Wechselwirkung mit geeigneten Lösungsmitteln bzw. Waschflüssigkeiten vergleichsweise einfach aus dem Produktgemisch entfernt werden, wobei insbesondere bekannte Verfahren zur Kohlendioxidentfernung, insbesondere entsprechende Wäschen (beispielsweise Aminwäschen) zum Einsatz kommen können. Kohlendioxid kann daher also bedarfsweise abgetrennt und in der zugehörigen Regenerierung nahezu als Reinsubstanz gewonnen werden. Eine solche Kohlendioxidentfernung kann an geeigneter Stelle des Prozesses, erfolgen. Eine weitere Feinreinigung und Reduzierung des Kohlendioxidgehaltes ist wie bereits zuvor erwähnt prinzipiell durch eine nachgeschaltete Laugewäsche möglich.

Zusätzlich offenbart die Erfindung, insbesondere in entsprechenden vorteilhaften Ausgestaltungen, eine vorteilhafte Wahl der Druckniveaus der entsprechenden Prozessschritte. Dabei werden, wie im Folgenden ausgeführt bedarfsweise auch weitere notwendige Verfahrensschritte wie Demethanizer und/oder C2-Splitter in vorteilhafter Weise einbezogen und berücksichtigt. Grundsätzlich können innerhalb des Gesamtprozesses an geeigneter Stelle auch Reaktions- und Verfahrensschritte angeordnet sein, um Nebenprodukte oder Spurenverbindungen zu entfernen. Insbesondere kann das erfindungsgemäße Verfahren hierzu notwendige Reinigungsschritte zur Entfernung von Spuren, insbesondere von Kohlenmonoxid, Acetylen und/oder Sauerstoff, enthalten.

In einer entsprechenden Gruppe von Ausgestaltungen bzw. einem weiteren Aspekt, die bzw. der weiter unten erläutert wird, adressiert die vorliegende Erfindung also das Problem, dass der Produktstrom der ODHE, wie zuvor erwähnt, neben Ethylen und Komponenten wie Ethan und Kohlendioxid und insbesondere auch Kohlenmonoxid und Acetylen (typischerweise in einem Gehalt von weniger als 300 bis 400 vppm) enthält. Dies betrifft insbesondere eine Gasphase des Produktstromes einer ODHE nach einer Kondensation.

Die erwähnte Aufgabe der Erfindung wird dadurch gelöst, dass die jeweiligen Gasphasen-Produktströme einer ODHE und einer Vinylacetatsynthese zumindest jeweils teilweise einer gemeinsamen Kohlendioxidentfernung unterworfen werden. Diese Kohlendioxidentfernung ist dabei bevorzugt als absorptive und regenerative Wäsche ausgeführt. Um eine möglichst effiziente Kohlendioxidentfernung zu realisieren, wird für die Kohlendioxidentfernung vorteilhafterweise ein erhöhter Druck gewählt. Im Rahmen der vorliegenden Erfindung erfolgt die Kohlendioxidentfernung daher vorzugsweise auf einem höheren Druckniveau als die ODHE und die Vinylacetatsynthese. Daher können die jeweiligen Gasphasen-Produktströme dieser Schritte (d.h. der ODHE und der Vinylacetatsynthese) einer gemeinsamen Verdichtung und danach der erfindungsgemäßen gemeinsamen Kohlendioxidentfernung zugeführt werden. Die gemeinsame Verdichtung kann ein- oder mehrstufig ausgeführt sein. Entsprechend kann die Zusammenführung beider Teilströme auch erst nach einer geeigneten Verdichterstufe erfolgen, d.h. dass zunächst in einer oder mehreren ersten Verdichterstufen nur der Produktstrom eines der beiden Schritte verdichtet wird und in einer oder mehreren zweiten Verdichterstufen dann die weitere gemeinsame Verdichtung erfolgt.

Das in einer absorptiven Wäsche entfernte Kohlendioxid kann bei Regeneration des Waschmittels sodann wieder als Kohlendioxidfraktion erhalten werden und in geeigneten Anteilen dem Feedstrom der Vinylacetatsynthese zugeführt werden, um hier eine bedarfsgerechte Verdünnung mit Kohlendioxid zu ermöglichen. Als besonderer Vorteil ergibt sich hier, dass die Kohlendioxidkonzentration am Eintritt der Vinylacetatsynthese unabhängig vom Kohlendioxidgehalt im Produktstrom der ODHE in einem sehr weiten Bereich eingestellt werden kann. Daher stellt diese Ausführungsform eine besonders vorteilhafte Variante dar.

Es kann prinzipiell auch die Zuführung eines Teilstroms des Produktgemischs der ODHE stromauf der Kohlendioxidentfernung in die Vinylacetatsynthese erfolgen. Eine solche Prozessführung ist grundsätzlich aus WO 2018/114747 A1 bekannt. Da ein solcher Teilstrom jedoch eine bestimmte Zusammensetzung insbesondere in Hinblick auf Kohlendioxid sowie Ethan und ggf. Methan (vgl. Ausführungen weiter unten) aufweist und zudem auch Kohlenmonoxid und Acetylen enthält, die bekannte Gifte für Katalysatoren für die Vinylacetatsynthese darstellen, erscheint eine solche Prozessführung nicht vorteilhaft oder aber nur für einen geringen Anteil im Verhältnis zum Gesamtfeedstrom der Vinylacetatsynthese möglich. Ferner sind hier Fluktuationen der Prozessgaszusammensetzung im Rahmen von Schwankungen im Betrieb der ODHE und/oder Vinylacetatsynthese zu erwarten, wodurch wiederum eine weitere unerwünschte Rückkopplung auf die Vinylacetatsynthese zu erwarten ist.

Die Erfindung schlägt zur Erreichung der genannten Ziele ein Verfahren zur Herstellung von Vinylacetat vor, bei dem in einem oder mehreren ersten Verfahrensschritten Ethan und Sauerstoff unter Erhalt eines Ethan, Ethylen, Wasser, Essigsäure und Kohlendioxid enthaltenden ersten Produktgemischs einer oxidativen katalytischen Dehydrierung (ODHE) unterworfen werden, und bei dem in einem oder mehreren zweiten Verfahrensschritten Ethylen und Essigsäure unter Erhalt eines Ethylen, Vinylacetat und Kohlendioxid enthaltenden gasförmigen zweiten Produktgemischs einer katalytischen Vinylacetatsynthese unterworfen werden, wobei zumindest ein Teil des Ethylens und zumindest ein Teil der Essigsäure, die in dem einen oder den mehreren zweiten Verfahrensschritten der katalytischen Umsetzung unterworfen werden, durch zumindest einen Teil des Ethylens und zumindest einen Teil der Essigsäure aus dem ersten Produktgemisch gebildet werden.

Erfindungsgemäß ist vorgesehen, dass, wie bereits zuvor mit anderen Worten ausgeführt, unter Verwendung zumindest eines Teils des ersten Produktgemischs ein Ethan, Ethylen und Kohlendioxid enthaltendes erstes Folgegemisch gebildet wird, und dass unter Verwendung zumindest eines Teils des zweiten Produktgemischs ein Ethylen und Kohlendioxid enthaltendes zweites Folgegemisch gebildet wird. Hierbei umfasst die Bildung des ersten Folgegemischs, zumindest einen Teil des ersten Produktgemischs unter Erhalt einer Wasser und Essigsäure enthaltenden Kondensatfraktion und des ersten Folgegemischs einer kondensativen Abscheidung von Wasser und Essigsäure zu unterwerfen und zumindest einen Teil der Essigsäure, die in dem einen oder den mehreren zweiten Verfahrensschritten der katalytischen Vinylacetatsynthese unterworfen wird, durch zumindest einen Teil der Essigsäure aus der Kondensatfraktion zu bilden.

Ferner ist vorgesehen, dass unter Verwendung zumindest eines Teils des ersten Folgegemischs und unter Verwendung zumindest eines Teils des zweiten Folgegemischs, genauer durch Zusammenführen von jeweils zumindest einem überwiegenden Teil des ersten Folgegemischs und des zweiten Folgegemischs, ein Ethan, Ethylen und Kohlendioxid enthaltendes drittes Folgegemisch gebildet wird, wobei die Bildung des zweiten Folgegemischs umfasst, zumindest einen Teil des zweiten Produktgemischs unter Erhalt einer Vinylacetat enthaltenden Vorproduktfraktion und des zweiten Folgegemischs einer Vinylacetatabtrennung zu unterwerfen.

Es ist außerdem vorgesehen, dass zumindest ein Teil des dritten Folgegemischs einem oder mehreren dritten Verfahrensschritten unterworfen wird, wobei der eine oder die mehreren dritten Verfahrensschritte eine gemeinsame Kohlendioxidentfernung umfassen, wobei in dem einen oder in den mehreren dritten Verfahrensschritten ein Ethan und Ethylen enthaltendes viertes Folgegemisch und eine Kohlendioxidfraktion gebildet werden, und wobei zumindest ein Teil des vierten Folgegemischs einer Trennung unterworfen wird, in der eine Ethanfraktion und eine Ethylenfraktion gebildet werden.

Zumindest ein Teil des Ethylens, das in dem einen oder den mehreren zweiten Verfahrensschritten der katalytischen Vinylacetatsynthese unterworfen wird, wird durch zumindest ein Teil des Ethylens aus der Ethylenfraktion gebildet, und/oder zumindest ein Teil des Ethans, das in dem einen oder den mehreren ersten Verfahrensschritten der oxidativen katalytischen Dehydrierung unterworfen wird, wird durch zumindest einen Teil des Ethans aus der Ethanfraktion gebildet.

Weitere Details und Vorteile der erfindungsgemäß vorgeschlagenen Maßnahmen wurden bereits ausführlich erläutert. Ein Eintrittsstrom in die Vinylacetatsynthese kann insbesondere einen Gehalt zwischen 0 und 30% Kohlendioxid aufweisen, und der oder zumindest einer der dritten Verfahrensschritte kann in einem Druckbereich durchgeführt werden, der oberhalb und insbesondere um wenigstens 1 bar, 2 bar, 5 bar oder 10 bar oberhalb eines Druckbereichs liegt, in dem der oder die ersten und zweiten Verfahrensschritte durchgeführt werden.

Grundsätzlich kann der entsprechende Produktstrom nach der erfindungsgemäßen gemeinsamen Kohlendioxidentfernung (also das vierte Folgegemisch stromab des oder der dritten Verfahrensschritte, oder ein entsprechender Teil hiervon) direkt der Vinylacetatsynthese (also dem einen oder den mehreren zweiten Verfahrensschritten) zugeführt werden. Insbesondere noch enthaltenes Ethan und/oder Methan wirken dabei als inertes Verdünnungsmittel in der Vinylacetatsynthese.

Allerdings stehen einer derartigen Ausführung in der Praxis noch im Gasstrom enthaltene Anteile von Kohlenmonoxid und/oder Acetylen entgegen, die in der Vinylacetatsynthese als Katalysatorgifte wirken können, falls sie nicht zuvor entfernt wurden. Daher ist im Rahmen eines integrierten Verfahrens eine weitere Aufreinigung, wie sie weiter unten beschrieben ist, vorteilhaft. Diese kann stromauf oder stromab der Kohlendioxidentfernung, also stromauf oder stromab des oder der dritten Verfahrensschritte erfolgen, wobei in ersterem Fall, der den erfindungsgemäßen Fall darstellt, die Bildung des ersten Folgegemischs die zumindest teilweise Entfernung von Kohlenmonoxid und/oder Acetylen umfasst und in letzterem Fall das vierte Folgegemisch einer zumindest teilweisen Entfernung von Kohlenmonoxid und/oder Acetylen unterworfen wird.

Für die nachfolgend genannten zusätzlichen Trennschritte ist jedoch entsprechend der gewählten Temperaturniveaus neben der erfindungsgemäßen Kohlendioxidentfernung ggf. auch eine Trocknung vorzuschalten, um Verlegungen durch Ausfrieren von Wasser bzw. Kohlendioxid zu vermeiden.

Wie ebenfalls zuvor bereits mit anderen Worten erläutert, umfasst die Kohlendioxidentfernung vorteilhafterweise eine regenerative adsorptive Kohlendioxidentfernung, in der eine Waschflüssigkeit eingesetzt wird, die bei der regenerativen absorptiven Kohlendioxidentfernung mit Kohlendioxid beladen wird, und aus der das Kohlendioxid unter Erhalt der Kohlendioxidfraktion ausgetrieben wird. Zumindest ein Teil der Kohlendioxidfraktion kann dabei als Verdünnungsmittel durch die einen oder die mehreren zweiten Verfahrensschritte, also die Vinylacetatsynthese, geführt werden, wie ebenfalls bereits erläutert.

Die Bildung des ersten Folgegemischs umfasst, wie erwähnt, zumindest einen Teil des ersten Produktgemischs unter Erhalt einer Wasser und Essigsäure enthaltenden (flüssigen) Kondensatfraktion und des ersten Folgegemischs einer kondensativen Abscheidung von Wasser und Essigsäure (nachfolgend auch kurz "Kondensatabscheidung") zu unterwerfen. Zumindest ein Teil der Essigsäure, die in dem einen oder den mehreren zweiten Verfahrensschritten der katalytischen Vinylacetatsynthese unterworfen wird, wird, wie ebenfalls erwähnt, dabei durch zumindest einen Teil der Essigsäure aus der Kondensatfraktion gebildet. Letztere kann dabei insbesondere in einer Kondensattrennung (auch als Essigsäureaufreinigung bezeichnet) gebildet werden, die der Kondensatabscheidung nachgeschaltet ist. Auch zu diesem Aspekt einer Ausgestaltung der vorliegenden Erfindung kann auf die obigen Erläuterungen verwiesen werden.

In einer Ausgestaltung der vorliegenden Erfindung kann die Bildung des dritten Folgegemischs umfassen, zumindest einen Teil des ersten Folgegemischs und zumindest einen Teil des zweiten Folgegemischs einer gemeinsamen Verdichtung zu unterwerfen. Wie auch diesbezüglich bereits erläutert, kann dabei beispielsweise ein mehrstufiger Verdichter bekannter Art eingesetzt werden, dem zumindest ein Teil des ersten Folgegemischs stromauf einer ersten Verdichterstufe und zumindest ein Teil des zweiten Folgegemischs stromauf einer zweiten Verdichterstufe zugeführt werden kann, wobei die zweite Verdichterstufe stromauf oder stromab der ersten Verdichterstufe angeordnet sein kann.

In dem erfindungsgemäß vorgeschlagenen Verfahren umfasst die Bildung des zweiten Folgegemischs, zumindest einen Teil des zweiten Produktgemischs unter Erhalt einer Vinylacetat enthaltenden Vorproduktfraktion und des zweiten Folgegemischs einer Vinylacetatabtrennung zu unterwerfen, wie ebenfalls bereits erwähnt. In der Vinylacetatabtrennung kann dabei ein Teil der Essigsäure aus der erwähnten Kondensatfraktion als Absorptionsmittel verwendet werden.

Zumindest ein Teil der Vorproduktfraktion kann im Rahmen der vorliegenden Erfindung einer Vinylacetatreinigung unterworfen werden, in der eine Vinylacetatfraktion, eine Nebenproduktfraktion und eine Essigsäurefraktion gebildet werden können. Hierbei kann insbesondere zumindest ein Teil der Essigsäure, die in dem einen oder den mehreren zweiten Verfahrensschritten der katalytischen Vinylacetatsynthese unterworfen wird, durch zumindest einen Teil der Essigsäure aus der Essigsäurefraktion aus der Vinylacetatreinigung gebildet werden.

Wie bereits erwähnt, wird zumindest ein Teil des vierten Folgegemischs einer Trennung unterworfen, in der eine Ethanfraktion und eine Ethylenfraktion gebildet werden. Die Trennung umfasst dabei insbesondere eine Trocknung an sich bekannter und oben beschriebener Art, gefolgt von einer erfindungsgemäßen Demethanisierung, und eine C2-Trennung in einem C2-Splitter. Aber auch andere Trennreihenfolgen und Trennanordnungen sind verwendbar. Zu Details sei auf die obigen Erläuterungen verwiesen. Zumindest ein Teil des Ethylens, das in dem einen oder den mehreren zweiten Verfahrensschritten der katalytischen Vinylacetatsynthese unterworfen wird, wird dabei durch zumindest ein Teil des Ethylens aus der Ethylenfraktion gebildet, und/oder zumindest ein Teil des Ethans, das in dem einen oder den mehreren ersten Verfahrensschritten der oxidativen katalytischen Dehydrierung unterworfen wird, wird dabei durch zumindest einen Teil des Ethans aus der Ethanfraktion gebildet. Auch zu diesem Aspekt wurden weitere Details zuvor erläutert.

In einer bevorzugten Ausgestaltung der Erfindung kann also ein C2-Splitter in das Verfahren integriert und dabei insbesondere der Feedstrom der Vinylacetatsynthese an Ethan abgereichert werden oder Ethan aus diesem Strom quantitativ entfernt werden. Insbesondere eine Ausführung, in dem die Ethylenfraktion aus dem C2-Splitter noch einen gewissen Anteil an Ethan enthält ist dabei besonders vorteilhaft, um den Aufwand (apparativ/Investkosten/Betriebskosten) dieser Trennung zu minimieren. Insbesondere kann dabei der Ethananteil im Strom zur Vinylacetatsynthese, also die Ethylenfraktion, die in diesem Fall ohne weitere trenntechnische Bearbeitung in den einen oder die mehreren zweiten Verfahrensschritte zur Vinylacetatsynthese geführt wird, zwischen 0 und 20 mol.-%, insbesondere zwischen 0,01 und 10 mol.-%, weiter insbesondere zwischen 0,5 und 5 mol.-% Ethan betragen. Dabei kann auch eine Gasphase aus der Vinylacetatsynthese, die entsprechend Ethan und nicht umgesetztes Ethylen enthält, an einer geeigneten Stelle stromauf des C2-Splitters zurückgeführt werden.

Über die erfindungsgemäße gemeinsame Kohlendioxidentfernung kann sich aufgrund der Rückführung eine gewisse Konzentration an Ethan im Kreislauf über die Vinylacetatsynthese aufbauen, wobei der gemäß der erläuterten Ausgestaltung vorgesehene C2-Splitter aber eine kontinuierliche Ausschleusung von Ethan bewirkt und eine zu hohe Ethanverdünnung vermeidet. Auch kann auf diese Weise eine optimale Ethanverdünnung in diesem Strom erreicht werden kann. Der Ethanstrom des C2-Splitters (die erläuterte Ethanfraktion) wird vorteilhafterweise zumindest teilweise zur ODHE zurückgeführt und enthält hingegen vorzugsweise einen möglichst geringen Anteil an Ethylen, jedoch ist auch hier keine quantitative Entfernung von Ethylen zwingend erforderlich. Der Gehalt an Ethylen kann beispielsweise zwischen 0 und 20 mol.-%, insbesondere zwischen 0,01 und 10 mol.-%, weiter insbesondere zwischen 0,5 und 5 mol.-% liegen.

Im Rahmen der vorliegenden Erfindung kann ein C2-Splitter vorteilhafterweise bereits bei relativ niedrigem Druck ab ca. 5 bar betrieben werden (vgl. auch Ausführungen oben). Für die technische Ausführung ist also insbesondere ein Druck, der wenig oberhalb der Vinylacetatsynthese liegt, vorteilhaft. Unter "wenig oberhalb" sei hier ein Druckunterschied von bis zu 15 bar, bevorzugt von bis zu 10 bar und besonders bevorzugt von bis zu 5 bar, verstanden.

Der in einer Ausgestaltung der Erfindung vorgesehene Demethanizer kann insbesondere zur zumindest teilweisen Entfernung insbesondere von Methan, aber insbesondere auch von Kohlenmonoxid, aus dem Prozessgasstrom, also aus dem vierten Folgegemisch oder einem weiteren Folgegemisch hiervon dienen bzw. eingerichtet sein, um z.B. eine Anreicherung in einem Kreislauf über die Vinylacetatsynthese zu vermeiden bzw. zu begrenzen. Der Demethanizer kann an geeigneter Stelle des Verfahrens angeordnet sein und insbesondere einem C2-Splitter vor- oder im Kopfstrom eines C2-Splitters nachgeschaltet sein. Der Kopfstrom eines Demethanizers kann neben Methan auch insbesondere Kohlenmonoxid und Sauerstoff enthalten, sofern diese nicht in einer stromaufliegenden Spurenentfernung - z.B. in einer unten erläuterten Rohgasbehandlung - entfernt werden. Wie zuvor beschrieben, erfordert die Gewährleistung eines Flüssigrücklaufs (Kondensation der C1minus-Fraktion) entsprechend niedrige Temperaturen. Nichtsdestotrotz kann ein Demethanizer - bei Einstellung einer entsprechend niedrigen Kopftemperatur - ebenfalls bei einem Druck, der wenig oberhalb der Vinylacetatsynthese liegt, im Rahmen der vorliegenden Erfindung vorteilhaft betrieben werden.

In einer besonders vorteilhaften Ausführung der vorliegenden Erfindung erfolgt hingegen stromauf des Demethanizers in einer an anderer Stelle auch als Spurenentfernung bezeichneten Rohgasbehandlung bzw. in Verfahrensschritten beliebiger Art eine vollständige oder zumindest überwiegende Entfernung von Kohlenmonoxids und es ist keine vollständige Abtrennung von Methan aus dem Prozessstrom im Demethanizer erforderlich, sondern eine Abreicherung ausreichend, also eine Reduzierung der im Prozessstrom enthaltenen Masse an Methan um mindestens 90%, bevorzugt mindestens 50% und besonders bevorzugt mindestens 25%. Der verbleibende Methananteil kann nach der Vinylacetatsynthese vergleichsweise leicht aus dem Produktstrom der Vinylacetatsynthese abgetrennt werden. Es ist dabei also wesentliche Aufgabe des Demethanizers, den Gehalt an Methan stromauf der Vinylacetatsynthese auf ein für die Vinylacetatsynthese geeignetes Niveau zu reduzieren und/oder eine Anreicherung von Methan in einem möglichen Kreislauf zu vermeiden. Im Gegensatz zur üblicherweise angestrebten Bereitstellung eines Reinethylenproduktes ist dabei eine quantitative oder nahezu quantitative Entfernung von Methan aus dem Ethylenfeed der Vinylacetatsynthese aber eben nicht erforderlich. Dies stellt einen weiteren besonderen Vorteil der vorliegenden Erfindung gegenüber üblichen Trenn- und Aufreinigungssequenzen dar. Dieser besondere Vorteil wird erst durch die vorgeschaltete Kohlenmonoxidentfernung (Rohgasbehandlung) ermöglicht.

Aspekte von Ausgestaltungen der Erfindung, die in beliebiger Weise mit sämtlichen erläuterten Ausgestaltungen und untereinander kombiniert werden können, umfassen also insbesondere, dass zumindest Methan und/oder Kohlenmonoxid mittels eines Demethanizers zumindest teilweise aus dem Prozessstrom entfernt werden und/oder dass die Kohlendioxidentfernung auf einem höheren Druckniveau als die ODHE und die Vinylacetatsynthese erfolgt und wobei stromauf der Kohlendioxidentfernung eine ein- oder mehrstufige Verdichtung erfolgt. Solche Aspekte umfassen ferner insbesondere eine zusätzliche Entfernung von Spuren, insbesondere von Kohlenmonoxid und/oder Acetylen. Die Spurenentfernung kann dabei in beliebiger Weise vorgesehen sein. Eine Entfernung von Kohlenmonoxid und/oder Acetylen kann vor oder nach einer Verdichtung und vor einer Kohlendioxidentfernung erfolgen. Insbesondere kann in sämtlichen Ausgestaltungen des erfindungsgemäßen Verfahrens der Austrittstrom aus einem ODHE-Reaktor (feuchtes Prozessgas, also das erste Produktgemisch) mehr als 0,40 mol.%, insbesondere mehr als 0,6 mol.% oder mehr als 1,5 mol.% Sauerstoff enthalten. Nach einer Entfernung von Kohlenmonoxid kann der Sauerstoffgehalt insbesondere mehr als 0,01 mol.%, mehr als 0,05 mol.% oder mehr als 0,20 mol.% betragen, insbesondere aber stets weniger als 0,4 mol.%. Die oxidative Dehydrierung kann insbesondere derart betrieben werden, dass im ersten Produktgemisch Ethylen und Essigsäure in einem Verhältnis zwischen 1 zu 1 und 10 zu 1 erzeugt werden, insbesondere in einem Verhältnis von 2 zu 1 bis 5 zu 1 und weiter insbesondere in einem Verhältnis von 2,5 zu 1 bis 4 zu 1.

In einer Gruppe von Ausgestaltungen, die bereits oben angesprochen wurde und nun weiter erläutert wird, adressiert die vorliegende Erfindung daher also das Problem, dass der Produktstrom der ODHE, wie zuvor erwähnt, neben Ethylen und Komponenten wie Ethan und Kohlendioxid insbesondere auch Kohlenmonoxid und Acetylen (typischerweise in einem Gehalt von weniger als 300 bis 400 vppm) enthält.

Dies betrifft insbesondere eine Gasphase des Produktstroms der ODHE, also des ersten Produktgemischs, nach einer Kondensation.

Wie erwähnt, sind insbesondere Acetylen und Kohlenmonoxid dabei aus dem Feedstrom der Vinylacetatsynthese möglichst vollständig zu entfernen, da beide potentielle Katalysatorgifte für die Vinylacetatsynthese sind. Vorhandene Ansätze zeigen keine vorteilhafte Lösung zur Entfernung dieser Spuren im Rahmen eines integrierten Verfahrens auf. Die vorliegende Erfindung offenbart also in Ausgestaltungen eine optimierte Lösung für eine Spurenentfernung (insbesondere von Acetylen und/oder Kohlenmonoxid) in einer integrierten Anordnung von ODHE und Vinylacetatsynthese. Die Erfindung macht sich dabei in einer ersten Ausführungsform (mit einer "Rohgasbehandlung", siehe oben) in geschickter Weise die folgenden Tatsachen zu Nutze:
1. Eine oxidative Entfernung von Acetylen und ggf. Kohlenmonoxid in der Gasphase des Austrittstromes einer ODHE ist bekannt.
2. Die ODHE benötigt einen Restgehalt an Sauerstoff am Reaktoraustritt. Die Gasphase des Austrittstromes der ODHE enthält also einen gewissen Gehalt an Sauerstoff.
3. Gleichzeitig wird für die Vinylacetatsynthese wie zuvor beschrieben Sauerstoff zugesetzt.

Eine gemeinsame katalytische Umsetzung von Sauerstoff und Acetylen in der Gasphase eines Austrittstroms aus einer ODHE über geeigneten Katalysatoren in einer solchen Rohgasbehandlung wird wie zuvor ausgeführt z.B. in WO 2020/187572 A1 und WO 2018/153831 A1 beschrieben. Dabei ist es jedoch das Ziel sowohl Sauerstoff als auch Acetylen nahezu quantitativ zu entfernen, um einerseits in der weiteren Produktaufreinigung insbesondere durch Fraktionierung ohne zusätzliche Maßnahmen die gewünschte Ethylenqualität zu erzielen und andererseits auch die Akkumulation von Sauerstoff in bestimmten Fraktionen, insbesondere in einer C1minus-Fraktion, zu vermeiden. Auch Kohlenmonoxid kann dabei zumindest teilweise umgesetzt werden.

Gegebenenfalls kann bei einer Integration einer ODHE und einer Vinylacetatsynthese in einer nicht erfindungsgemäßen Ausgestaltung hier auch keine vollständige Sauerstoffentfernung vorgesehen sein, bevor das Produktgemisch der ODHE (zuvor auch als "erstes Produktgemisch" bezeichnet) als Quelle für Ethylen und Essigsäure der Vinylacetatsynthese zugeführt wird. Verbleibender Sauerstoff kanndann in die Vinylacetatsynthese überführt werden. Er dient dort als Reaktionspartner. Es kann somit zunächst ein erhöhter Sauerstoffgehalt am Austritt der ODHE zugelassen werden, was besonders vorteilhaft für den Betrieb der ODHE ist, die einen Mindestsauerstoffanteil im Austrittsstrom erfordert.

Durch einen erhöhten Sauerstoffanteil kann dabei insbesondere eine höhere Toleranz der ODHE gegenüber etwaigen Betriebsschwankungen und eine insgesamt längere Katalysatorstandzeit erreicht werden. Bedarfsweise kann auch eine zusätzliche Einspeisung von Sauerstoff und/oder Wasserstoff vor oder während der Rohgasbehandlung erfolgen (vgl. WO 2020/187572 A1). Hierbei ist nun als besonderer Vorteil auch der Sauerstoffgehalt im Eintritt der Rohgasbehandlung ein zusätzlicher Freiheitsgrad. Neben dem Sauerstoff aus dem Austritt der ODHE kann also bedarfsweise zielgerichtet weiterer Sauerstoff vor der Rohgasbehandlung eingespeist werden und somit eine optimale Performance dieser Rohgasbehandlung zur Acetylen- und/oder Kohlenmonoxidentfernung erreicht werden. Entsprechend kann die weitere Einspeisung von Sauerstoff in den Feed der Vinylacetatsynthese reduziert werden. In der Rohgasbehandlung können prinzipiell dem Fachmann bekannte Katalysatoren eingesetzt werden.

Dabei kommen insbesondere die in WO 2020/187572 A1 und WO 2018/153831 A1 beschriebenen Katalysatoren zum Einsatz. Daher wird an dieser Stelle entsprechend auf diese beiden Schriften und die dort enthaltenen weiteren Ausführungen verwiesen. Insbesondere können in der Rohgasbehandlung Kupferoxid enthaltende Katalysatoren oder zumindest eines der Elemente Kupfer, Mangan, Zink, Nickel, Platin, Palladium, Rhodium und/oder Ruthenium enthaltende Katalysatoren verwendet werden. Eine solche Rohgasbehandlung kann erfindungsgemäß vor oder nach einer Verdichtung der Gasphase des Austrittsstromes der ODHE angeordnet sein oder auch auf einem geeigneten Druckniveau zwischen zwei Verdichterstufen Mit anderen Worten wird der Rohgasbehandlung das zuvor als "erstes Produktgemisch" bezeichnete Komponentengemisch zugeführt.

In der Gasphase der ODHE enthaltenes Ethan und ggf. Methan können ebenfalls ohne Abtrennung der Vinylacetatsynthese zugeführt werden und dienen dort als Inert- bzw. Verdünnungsmedium. In Ausgestaltungen können sodann weitere Verfahrensschritte einbezogen werden, die wie weiter unten ausgeführt neben einer Kohlendioxidentfernung, wie zuvor ausführlich gewürdigt, in geeigneter Anordnung insbesondere eine Trocknung, einen optionalen Demethanizer und/oder einen C2-Splitter umfassen können.

Nicht umgesetztes Ethylen kann sodann also wieder zur Vinylacetatsynthese vollständig oder teilweise rezykliert werden. Neben Ethylen können in diesem Restgasstrom auch andere Komponenten, insbesondere Kohlendioxid, Ethan und/oder Methan enthalten sein. Sofern die Rohgasbehandlung dabei keine ausreichende Kohlenmonoxidentfernung erreicht bzw. in einer Weise betrieben wird, dass nur Acetylen ausreichend entfernt, kann verbleibendes Kohlenmonoxid im Prozesstrom nach der Rohgasbehandlung bedarfsweise in einem Demethanizer entfernt werden, der ebenfalls zur zumindest teilweisen Entfernung von Methan fungiert. Sofern der Restgasstrom der Vinylacetatsynthese insbesondere keine Anteile an insbesondere Acetylen und/oder ggf. Kohlenmonoxid enthält, kann der oben dargelegte Recycle ohne Einbezug der Rohgasbehandlung erfolgen.

Sofern hingegen auch der Restgasstrom aus der Vinylacetatsynthese Anteile an insbesondere Acetylen und/oder ggf. Kohlenmonoxid enthält, kann in einer Ausgestaltung der vorliegenden Erfindung jedoch auch vorteilhafterweise die Rohgasbehandlung in den oben dargelegten Recycle einbezogen werden. Dazu erfolgt eine zumindest teilweise Einspeisung des Restgasstromes aus der Vinylacetatsynthese in die Gasphase des Austrittsstromes der ODHE. Auf diese Weise wird dann auch der Restgasstrom aus einer Vinylacetatsynthese nach Abtrennung kondensierbarer Anteile einer gemeinsamen Rohgasbehandlung unterworfen werden. Es sind somit keine zusätzlichen Reinigungsstufen im Restgasstrom der Vinylacetatsynthese erforderlich, Auch in diesen Fällen kann die Rohgasbehandlung erfindungsgemäß vor oder nach einer Verdichtung der Gasphase des Austrittsstromes der ODHE angeordnet sein oder auch auf einem geeigneten Druckniveau zwischen zwei Verdichterstufen. Eine zumindest teilweise Einspeisung des Restgasstromes aus der Vinylacetatsynthese erfolgt an geeigneter Stelle stromauf der Rohgasbehandlung.

In einer weiteren Ausführungsform ("Tailend-Hydrierung") macht sich die Erfindung in geschickter Weise die folgenden Tatsachen zu Nutze:
1. Die Entfernung von Acetylen aus ethylenreichen Prozesströmen über eine so genannte Tailend-Hydrierung ist bekannt.
2. Eine Entfernung von Kohlenmonoxid kann in einem Demethanizer über die C1minus-Fraktion erfolgen.
3. Eine Sauerstoffentfernung ist ebenfalls über die C1minus Fraktion in einem Demethanizer möglich.
4. Alternativ ist auch eine eigenständige oxidative Umsetzung von CO mit Sauerstoff bekannt.

Es erfolgt zunächst eine zumindest teilweise Vereinigung der Gasphase des Austrittstromes einer ODHE und des Restgasstromes einer Vinylacetatsynthese. Das vereinigte Gasgemisch durchläuft sodann geeignete Verfahrensschritte, die - wie ähnlich bereits für die Rohgasbehandlung und im Detail weiter unten ausgeführt - in geeigneter Anordnung insbesondere eine Kohlendioxidentfernung, eine Trocknung, einen Demethanizer und/oder einen C2-Splitter umfassen können. Dabei dient insbesondere der Demethanizer nun dazu, in dem vereinigten Gasgemisch enthaltenes Kohlenmonoxid zu entfernen. Zugleich werden vorhandene Anteile von Methan und/oder Sauerstoff entfernt. In dieser Ausführung ist die Entfernung von Kohlenmonoxid und Kohlendioxid vorteilhaft, da diese beiden Verbindungen einen negativen Einfluss auf die Aktivität und/oder Selektivität in der nachfolgenden Tailend-Hydrierung haben. Die Entfernung von Acetylen erfolgt erfindungsgemäß sodann in einer nachgeschalteten Tailend-Hydrierung, die sich alternativ entweder stromauf oder stromab eines C2-Splitters befinden kann. Auf diese Weise können im Endergebnis also ebenfalls Spuren von Kohlenmonoxid und/oder Acetylen in einer optimierten Verfahrenskette entfernt werden und es sind wiederum keine zusätzlichen Reinigungsstufen im Restgasstrom der VAM-Synthese für diese Aufgabe(n) notwendig. In der Tailend-Hydrierung können prinzipiell dem Fachmann bekannte Katalysatoren eingesetzt werden. Dabei können insbesondere Katalysatoren, die zumindest eines der Elemente Ni, Pd, Rh, Ir, Ru, Pt, Ce, Ag und Au enthalten, eingesetzt werden.

Eine Anlage zur Herstellung von Vinylacetat ist ebenfalls Gegenstand der vorliegenden Erfindung. Zu den Merkmalen einer derartigen Anlage sei auf den entsprechenden unabhängigen Patentanspruch ausdrücklich verwiesen.

Zu Merkmalen und Vorteilen der erfindungsgemäß vorgeschlagenen Anlage und vorteilhafter Ausgestaltungen hiervon sei ferner auf die obigen Ausführungen bezüglich des erfindungsgemäßen Verfahrens und vorteilhafter Ausgestaltungen hiervon ausdrücklich verwiesen. Insbesondere ist die erfindungsgemäß vorgeschlagene Anlage bzw. sind entsprechende Ausgestaltungen dazu eingerichtet, entsprechende Verfahrensvarianten durchzuführen.

### Ausführungsbeispiele

Die Erfindung wird nachfolgend anhand von Beispielen, die Ausgestaltungen der Erfindung entsprechen, und zugehörigen Figuren weiter erläutert.

Die Figuren 1 bis 4 veranschaulichen Verfahren und Anlagen, anhand derer Merkmale erfindungsgemäßer Ausgestaltungen erläutert werden, in Form schematischer Prozessdiagramme, wobei Figur 2 sich auf eine nicht erfindungsgemäße Variante bezieht.

Wird nachfolgend auf Aspekte eines Verfahrens Bezug genommen, betreffen diese Erläuterungen entsprechende Anlagen und ihre Ausgestaltungen in gleicher Weise. Entsprechendes gilt für Verfahrensschritte und Anlagenkomponenten. Verfahrensschritte und Anlagenkomponenten gleicher oder vergleichbarer Funktion und/oder technischer Realisierung sind mit identischen Bezugszeichen angegeben.

Das in Figur 1 veranschaulichte Verfahren ist insgesamt mit 100 bezeichnet. In dem Verfahren 100 werden einer ODHE (zuvor als ein oder mehrere erste Verfahrensschritte bzw. Prozesskomponenten 10 bezeichnet) die benötigten Reaktanden Ethan C₂H₆ und Sauerstoff O₂ zugeführt. Der Ethanstrom C₂H₆ kann dabei in Abhängigkeit von der Ethanquelle und evtl. vorgeschalteten Reinigungsschritten auch gewisse Anteile z.B. von Methan und/oder höheren Kohlenwasserstoffen enthalten. Zusätzlich kann bedarfsweise Wasserdampf H₂O als Verdünnungsmittel eingespeist werden. Das dafür benötigte Wasser kann dabei insbesondere auch zumindest anteilig aus der Raffinatwasserphase einer optionalen Essigsäureaufreinigung bzw. Kondensattrennung 50 stammen, wie mit einem gestrichelten Pfeil veranschaulicht.

In der ODHE, also in dem einen oder den mehreren ersten Verfahrensschritte bzw. Prozesskomponenten 10 wird ein Produktgemisch gebildet, das zuvor als erstes Produktgemisch bezeichnet wurde und hier mit 11 angegeben ist. Nach einer Abkühlung und Kondensation (nicht dargestellt) erfolgt eine Phasentrennung bzw. Abscheidung 40 einer Kondensatfraktion 41, also einer wässrigen Phase, die Essigsäure enthalten kann. Diese kann der erwähnten Essigsäureaufreinigung bzw. Kondensattrennung 50 zugeführt werden. Die verbleibende Gasphase, die im Wesentlichen nicht umgesetztes Ethan sowie Ethylen und Kohlendioxid sowie gewisse Anteile von Kohlenmonoxid und ggf. weiteren Spurenverbindungen enthält, zuvor auch als erstes Folgegemisch bezeichnet, wird sodann einer Verdichtung 45 auf ein benötigtes Druckniveau verdichtet und einer Kohlendioxidentfernung zugeführt, die hier Teil eines oder mehrerer Verfahrensschritte bzw. entsprechender Anlagenkomponenten sein kann, die zuvor als dritte Verfahrensschritte bzw. Anlagenkomponenten 30 bezeichnet wurden.

Dabei erfolgt die Einspeisung eines Rückführstroms 22 aus einer Vinylacetatsynthese, die hier Teil eines oder mehrerer Verfahrensschritte bzw. entsprechender Anlagenkomponenten sein kann, welche zuvor als zweite Verfahrensschritte bzw. Anlagenkomponenten 20 bezeichnet wurden, und einer anschließenden Wäsche 60 entsprechend dem Druckniveau vor der Verdichtung 45 bzw. vor einer entsprechenden Verdichterstufe. Der Rückführstrom 22 wurde zuvor auch als zweites Folgegemisch bezeichnet.

Gemäß der hier veranschaulichten Ausgestaltung werden also sowohl der gasförmige Anteil des Produktes der ODHE (erstes Folgegemisch 12) als auch der gasförmige Reststrom aus der Vinylacetatsynthese (zweites Folgegemisch 22) einer gemeinsamen Kohlendioxidentfernung in dem oder den dritten Verfahrensschritten bzw.

Anlagenkomponenten 30 unterworfen. Ein entsprechend dort eingespeister und aus der Kombination des ersten und zweiten Folgegemischs (12 und 22) resultierender Stoffstrom wurde zuvor auch als drittes Folgegemisch 31 bezeichnet. Die Kohlendioxidentfernung umfasst dabei insbesondere eine absorptive Wäsche. Eine bei der Regeneration des beladenen Absorbens (nicht explizit dargestellt) gewonnene Kohlendioxidfraktion 33 kann - wenn gewünscht - teilweise der Vinylacetatsynthese als Verdünnungsmittel in einer bedarfsgerechten und jederzeit leicht einstellbaren Menge in Form eines Stoffstroms 34 zugeführt werden. Die Kohlendioxidentfernung kann dabei je nach Erfordernis auch eine optionale Laugewäsche (nicht explizit dargestellt) umfassen, die zur weiteren Reduzierung des Kohlendioxidgehalts in dem verbleibenden Gasstrom bzw. vierten Folgegemisch 32 mit einem bereits in der absorptiven Wäsche reduzierten Kohlendioxidgehalt dient.

Nach einer Trocknung 80 wird dieser Stoffstrom 32, zuvor auch als viertes Folgegemisch bezeichnet, weiteren destillativen Verfahrensschritten und einer weiteren Auftrennung zugeführt. Zu diesen destillativen Verfahrensschritte zählt insbesondere ein C2-Splitter bzw. eine C2-Trennung 90, der bzw. die den Prozessstrom insbesondere in eine an Ethan zumindest angereicherte Fraktion (kurz "Ethanfraktion") C₂H₆ sowie in eine an Ethylen zumindest angereicherte Fraktion (kurz "Ethylenfraktion") C₂H₄ auftrennt. Des Weiteren kann aber auch ein optionaler Demethanizer 85 in der Prozessanordnung enthalten sein. Dieser optionale Demethanizer 85 dient dabei zur Entfernung von leichten Komponenten als C1minus Fraktion, die insbesondere die Komponenten Methan, Kohlenmonoxid und Sauerstoff beinhalten kann. Der optionale Demethanizer 85 kann wahlweise wie in Figur 1 dargestellt stromauf des C2-Splitters 90, oder aber stromab eines entsprechenden C2-Splitters in der an Ethylen angereicherten Fraktion bzw. Ethylenfraktion C₂H₄ angeordnet sein. Die an Ethan zumindest angereicherte Fraktion bzw. Ethanfraktion C₂H₆ wird wiederum der ODHE bzw. dem einen oder den mehreren ersten Verfahrensschritten 10 als Einsatzstrom zugeführt. Die an Ethylen zumindest angereicherte Fraktion bzw. Ethylenfraktion C₂H₄ wird der Vinylacetatsynthese bzw. dem einen oder den mehreren zweiten Verfahrensschritten 20 als Einsatzstrom zugeführt. Optional kann auch ein Teilstrom dieser Fraktion C₂H₄ abgezweigt und für andere Prozesse als Eduktstrom bereitgestellt werden (nicht explizit dargestellt).

Weitere Einsatzströme der Vinylacetatsynthese bzw. des einen oder der mehreren zweiten Verfahrensschritte 20 umfassen dabei insbesondere eine Zuführung von Sauerstoff O₂, eine Bereitstellung von Essigsäure insbesondere aus der zuvor genannten Essigsäureaufreinigung bzw. Kondensattrennung 50, sowie die bereits erwähnte bedarfsgerechte Bereitstellung von Kohlendioxid aus der Kohlendioxidentfernung 30 bzw. dem einen oder den mehreren dritten Verfahrensschritten. Dabei kann zusätzlich auch eine Rückführung einer Essigsäurefraktion aus einer Vinylacetataufbereitung 70, die insbesondere destillative Verfahren beinhalten kann, in Form eines Stoffstroms 73, sowie ggf. auch aus einer zusätzlichen externen Quelle, wie mit C₂H₄O₂ bezeichnet, erfolgen.

Der Austrittsstrom aus der der Vinylacetatsynthese bzw. des einen oder der mehreren zweiten Verfahrensschritte 20 wird sodann der bereits beschriebenen Wäsche 60 unterzogen, die einerseits einen Gasstrom erzeugt, der, wie beschrieben, als zweites Folgegemisch 22 der Verdichtung 45 zugeführt wird und der insbesondere nicht umgesetztes Ethylen sowie Kohlendioxid beinhaltet. Zusätzliches Kohlendioxid entsteht dabei als Nebenprodukt der Vinylacetatsynthese. Weitere flüchtige Bestandteile können ebenfalls enthalten sein, z.B. Sauerstoff oder Bestandteile aus der an Ethylen angereicherten Fraktion bzw. Ethylenfraktion C₂H₄ aus dem C2-Splitter, insbesondere Ethan. Der Wäsche 60 wird als Waschmittel ebenfalls ein Essigsäurestrom zugeführt, der entweder aus der Essigsäureaufreinigung bzw. Kondensattrennung 50 und/oder der bereits erwähnten insbesondere destillativen Vinylacetatreinigung 70 und/oder einer insbesondere zusätzlichen externen Quelle stammen kann. Mit diesem Waschmittel wird sodann das Reaktionsprodukt Vinylacetat aus dem (zweiten) Produktgemisch der Vinylacetatsynthese bzw. des einen oder der mehreren zweiten Verfahrensschritte 20 entfernt und der bereits erwähnten insbesondere destillativen Vinylacetataufreinigung 70 zugeführt. In dem Verfahrensschritt 70 wird dann das gewünschte Endprodukt Vinylacetat in Form einer Vinylacetatfraktion 71 in der benötigten Reinheit gewonnen. Es fällt auch eine Nebenproduktfraktion 72 an, die insbesondere Wasser, aber auch andere Nebenprodukte der Vinylacetatsynthese enthält.

Der Prozess 100 kann zudem bedarfsweise weitere optionale Reinigungsschritte zur Entfernung von Spurenverbindungen und optionale Purge-Ströme zur Vermeidung der Akkumulation von unerwünschten Spurenverbindungen in Recycle-Strömen beinhalten. Diese sind in Figur 1 nicht explizit dargestellt. Es wird auf die Figuren 3 und 4 verwiesen.

In Figur 2 ist ein weiteres Verfahren bzw. eine weitere Anlage in Form eines schematischen Ablaufplans veranschaulicht und insgesamt mit 200 bezeichnet. Es gelten grundsätzlich die Erläuterungen zu Figur 1. Abweichungen und Ergänzungen sind im Folgenden erläutert.

Eine in der Vinylacetatabtrennung 60 verbleibende Gasphase, die auch hier mit 22 bezeichnet ist, wird hier zunächst einer weiteren Verdichtung 45a unterworfen und dann einer Kohlendioxidentfernung bzw. entsprechenden Verfahrensschritten oder Anlagenkomponenten 30 zugeführt. Die Kohlendioxidentfernung umfasst dabei wie zuvor insbesondere eine absorptive Wäsche. Der in der Regeneration des beladenen Absorbens (nicht explizit dargestellt) gewonnene Kohlendioxidstrom 33 kann wie zuvor beschrieben - wenn gewünscht - teilweise der Vinylacetatsynthese bzw. dem oder den Verfahrensschritten bzw. Anlagenkomponenten 20 in Form eines Stoffstroms 34 als Verdünnungsmittel in einer bedarfsgerechten und jederzeit leicht einstellbaren Menge zugeführt werden. Kohlendioxidentfernung kann dabei je nach Erfordernis auch eine optionale Laugewäsche (nicht explizit dargestellt) umfassen, die zur weiteren Reduzierung des Kohlendioxidgehaltes in dem Gasstrom mit einem bereits in der absorptiven Wäsche reduzierten Kohlendioxidgehalt dient. Nach einer Trocknung 80 wird dieser Strom weiteren destillativen Verfahrensschritten und einer weiteren Auftrennung zugeführt. Zu diesen destillativen Verfahrensschritte zählen insbesondere ein C2-Splitter 90 und ein optionaler Demethanizer 85 wie bereits zu Figur 1 ausgeführt.

Im Unterschied zu der in Figur 1 dargestellten Ausgestaltung wird das erste Produktgemisch 11 bzw. ein entsprechendes Folgegemisch 22 hier nicht mit dem zweiten Produktgemisch 21 bzw. dem entsprechenden Folgegemisch 22 vereinigt, bevor beide dem oder den Verfahrensschritten bzw. Anlagenkomponenten 30, also der Kohlendioxidentfernung, zugeführt werden. Stattdessen erfolgt hier eine Einspeisung eines Folgegemischs 12a nach einer Verdichtung 45 in eine Spurenkomponentenentfernung 210, die insbesondere eine Acetylenhydrierung, eine Kohlenmonoxidelimination und/oder eine Sauerstoffentfernung umfassen kann, und in die ein Stoffstrom 211, beispielsweise umfassend zusätzlichen Wasser- und/oder Sauerstoff, eingespeist werden kann. Ein hier gebildetes Folgegemisch, mit 31 bezeichnet, wird dem oder den Verfahrensschritten bzw. Anlagenkomponenten 20, d.h. der Vinylacetatsynthese, zugeführt, und ein hier gebildetes Produktgemisch wird wie erläutert behandelt.

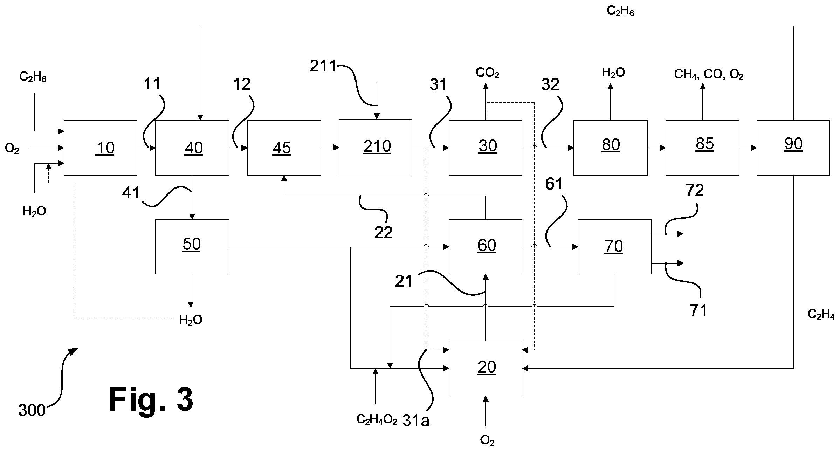

In Figur 3 ist ein weiteres Verfahren bzw. eine weitere Anlage in Form eines schematischen Ablaufplans veranschaulicht und insgesamt mit 300 bezeichnet. Es gelten grundsätzlich die Erläuterungen zu den Figuren 1 und 2 und das Verfahren bzw. die Anlage 100 bzw. 200 weiter. Abweichungen und Ergänzungen sind jeweils im Folgenden erläutert.

Die weitere Verdichtung 45a aus Figur 2 entfällt hier, und vergleichbar mit Figur 2 werden das erste und zweite Folgegemisch 12, 22 vor oder in der Verdichtung 45 zusammengeführt. Die Bildung eines dritten Folgegemischs 31 umfasst hier aber zusätzlich die bereits zu Figur 2 erläuterte Spurenkomponentenentfernung. Diese kann insbesondere wie in Figur 3 schematisch dargestellt und wie zuvor erläutert als Rohgasbehandlung ausgeführt sein. Auf diese Weise können die Gehalte an Kohlenmonoxid und/oder Acetylen auf ein für die Vinylacetatsynthese in dem oder den Verfahrensschritten bzw. Anlagenkomponenten 20 unkritisches Maß reduziert werden. Ein gestrichelt dargestellter Teilstrom 31a kann stromauf des oder der Verfahrensschritte bzw. Anlagenkomponenten 30 abgezweigt und direkt zur Vinylacetatsynthese geführt werden und die enthaltenen Anteile an Ethan und Kohlendioxid dienen hier wiederum als erwünschtes und in der Praxis notwendiges Verdünnungsmittel. Die weitere Fraktionierung entspricht im Wesentlichen der Prozessführung gemäß Figur 2, allerdings kann aufgrund der Positionierung Spurengasentfernung 210 nun eine deutliche Reduzierung des Sauerstoffanteiles im vierten Folgegemisch 32 erreicht werden. Hierdurch kann insbesondere eine übermäßige Anreicherung von Sauerstoff im Kopfstrom des optionalen Demethanizers 85 und die mögliche Bildung einer gefährlichen explosionsfähigen Atmosphäre in diesem Bereich vermieden werden.

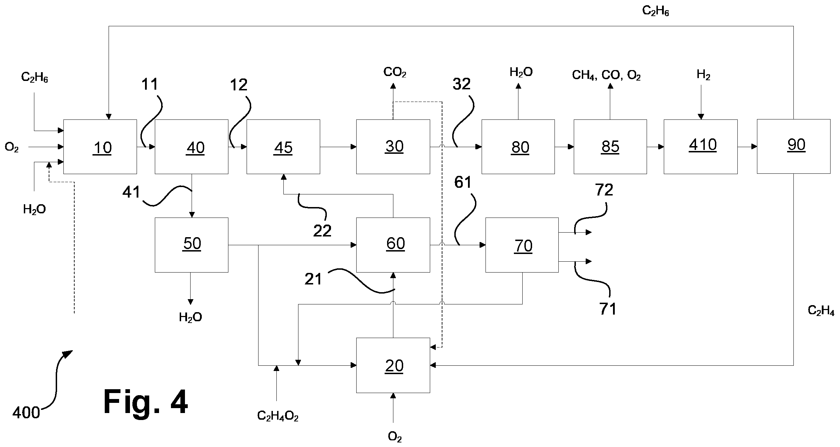

In Figur 4 ist ein weiteres Verfahren bzw. eine weitere Anlage in Form eines schematischen Ablaufplans veranschaulicht und insgesamt mit 400 bezeichnet. Es gelten grundsätzlich die Erläuterungen zu den Figuren 1 bis 3 und das Verfahren bzw. die Anlage 100 bis 300 weiter. Abweichungen und Ergänzungen sind jeweils im Folgenden erläutert.

Die Rohgasentfernung 210 aus Figur 3 entfällt. Entsprechend ist die Führung eines Teilstromes 31a stromauf der Kohlendioxidentfernung bzw. den Verfahrensschritten bzw. Anlagenkomponenten 30 direkt zur Vinylacetatsynthese bzw. den Verfahrensschritten bzw. Anlagenkomponenten 20 aufgrund der zu erwartenden Gehalte an Kohlenmonoxid und Acetylen an dieser Stelle technisch nicht sinnvoll. Die Entfernung von Kohlenmonoxid und Sauerstoffspuren erfolgt sodann mittels des Demethanizers 85 der also in dieser Ausgestaltung der Erfindung notwendigerweise vorzusehen ist. Aufgrund der bereits erwähnten möglichen Bildung einer gefährlichen explosionsfähigen Atmosphäre im Bereich des Kopfstromes des Demethanizers 85 sind besondere zusätzliche Aspekte bei der technischen Auslegung in diesem Bereich zu beachten gemäß den entsprechenden Regelwerken und dem Fachmann bekannten Vorgehensweisen im Rahmen des technischen Explosionsschutzes.

Der Feedstrom des C2-Splitters 90 wird in dieser Ausgestaltung der Erfindung nun einer Tailend-Hydrierung 410 unterworfen, die unter Zusatz von benötigtem Wasserstoff Acetylen selektiv zu Ethylen hydriert. Sowohl der Demethanizer 85 als auch die Tailend-Hydrierung 410 können jeweils unabhängig voneinander wahlweise wie in Figur 4 dargestellt stromauf des 90 oder aber stromab des C2-Splitters 90 in der an Ethylen angereicherten Fraktion C₂H₄ angeordnet sein. Dabei muss jedoch in jedem Fall der Demethanizer 85 stromauf der Tailend-Hydrierung 410 angeordnet sein, da Kohlenmonoxid und Sauerstoff in der Tailend-Hydrierung 410 jeweils einen negativen Einfluss haben.

## Patentansprüche

1. Verfahren zur Herstellung von Vinylacetat, bei dem in einem oder mehreren ersten Verfahrensschritten (10) Ethan und Sauerstoff unter Erhalt eines Ethan, Ethylen, Wasser, Essigsäure und Kohlendioxid enthaltenden ersten Produktgemischs (11) einer oxidativen katalytischen Dehydrierung unterworfen werden, und bei dem in einem oder mehreren zweiten Verfahrensschritten (20) Ethylen und Essigsäure unter Erhalt eines Ethylen, Vinylacetat und Kohlendioxid enthaltenden gasförmigen zweiten Produktgemischs (21) einer katalytischen Vinylacetatsynthese unterworfen werden, wobei zumindest ein Teil des Ethylens und zumindest ein Teil der Essigsäure, die in dem einen oder den mehreren zweiten Verfahrensschritten (20) der katalytischen Umsetzung unterworfen werden, durch zumindest einen Teil des Ethylens und zumindest einen Teil der Essigsäure aus dem ersten Produktgemisch (11) gebildet werden, **dadurch gekennzeichnet,**
- **dass** unter Verwendung zumindest eines Teils des ersten Produktgemischs (11) ein Ethan, Ethylen und Kohlendioxid enthaltendes erstes Folgegemisch (12) gebildet wird, wobei die Bildung des ersten Folgegemischs (12) eine zumindest teilweise Entfernung von Sauerstoff, Kohlenmonoxid und/oder Acetylen umfasst, und ferner umfasst, zumindest einen Teil des ersten Produktgemischs (11) unter Erhalt einer Wasser und Essigsäure enthaltenden Kondensatfraktion (41) und des ersten Folgegemischs (12) einer kondensativen Abscheidung (40) von Wasser und Essigsäure zu unterwerfen und wobei zumindest ein Teil der Essigsäure, die in dem einen oder den mehreren zweiten Verfahrensschritten (20) der katalytischen Vinylacetatsynthese unterworfen wird, durch zumindest einen Teil der Essigsäure aus der Kondensatfraktion (41) gebildet wird,
- **dass** unter Verwendung zumindest eines Teils des zweiten Produktgemischs (21) ein Ethylen und Kohlendioxid enthaltendes zweites Folgegemisch (22) gebildet wird, wobei die Bildung des zweiten Folgegemischs (22) umfasst, zumindest einen Teil des zweiten Produktgemischs (21) unter Erhalt einer Vinylacetat enthaltenden Vorproduktfraktion (61) und des zweiten Folgegemischs (22) einer Vinylacetatabtrennung (60) zu unterwerfen,
- **dass** durch Zusammenführen von jeweils zumindest einem überwiegenden Teil des ersten Folgegemischs (12) und des zweiten Folgegemischs (22) ein Ethan, Ethylen und Kohlendioxid enthaltendes drittes Folgegemisch (31) gebildet wird,
- **dass** zumindest ein Teil des dritten Folgegemischs (31) einem oder mehreren dritten Verfahrensschritten (30) unterworfen wird, wobei der eine oder die mehreren dritten Verfahrensschritte (30) eine Kohlendioxidentfernung umfassen, wobei in dem einen oder in den mehreren dritten Verfahrensschritten (30) ein Ethan und Ethylen enthaltendes viertes Folgegemisch (32) und eine Kohlendioxidfraktion (33) gebildet werden, und wobei zumindest ein Teil des vierten Folgegemischs (32) einer Trennung (85, 90) unterworfen wird, in der eine Ethanfraktion und eine Ethylenfraktion gebildet werden,
- **dass** zumindest ein Teil des Ethylens, das in dem einen oder den mehreren zweiten Verfahrensschritten (20) der katalytischen Vinylacetatsynthese unterworfen wird, durch zumindest ein Teil des Ethylens aus der Ethylenfraktion gebildet wird, und/oder zumindest ein Teil des Ethans, das in dem einen oder den mehreren ersten Verfahrensschritten (20) der oxidativen katalytischen Dehydrierung unterworfen wird, durch zumindest einen Teil des Ethans aus der Ethanfraktion gebildet wird, und
- **dass** zumindest ein Teil des vierten Folgegemischs und/oder zumindest ein Teil der Ethylenfraktion einer Demethanisierung (85) unterworfen wird.

2. Verfahren nach Anspruch 1, bei dem die Kohlendioxidentfernung eine regenerative adsorptive Kohlendioxidentfernung umfasst, in der eine Waschflüssigkeit eingesetzt wird, die bei der regenerativen absorptiven Kohlendioxidentfernung mit Kohlendioxid beladen wird, und aus der das Kohlendioxid unter Erhalt der Kohlendioxidfraktion (33) ausgetrieben wird.

3. Verfahren nach einem der vorstehenden Ansprüche, bei dem zumindest ein Teil der Kohlendioxidfraktion (33) als Verdünnungsmittel durch die einen oder die mehreren zweiten Verfahrensschritte (20) geführt wird.

4. Verfahren nach einem der vorstehenden Ansprüche, bei dem die Bildung des dritten Folgegemischs (31) umfasst, zumindest einen Teil des ersten Folgegemischs (12) und zumindest einen Teil des zweiten Folgegemischs (22) einer gemeinsamen Verdichtung (45) zu unterwerfen.

5. Verfahren nach einem der vorstehenden Ansprüche, bei dem in der Vinylacetatabtrennung (60) ein Teil der Essigsäure aus der Kondensatfraktion (41) als Absorptionsmittel verwendet wird.

6. Verfahren nach einem der vorstehenden Ansprüche, bei dem zumindest ein Teil der Vorproduktfraktion (61) einer Vinylacetatreinigung (70) unterworfen wird, in der eine Vinylacetatfraktion (71), eine Nebenproduktfraktion (72) und eine Essigsäurefraktion (73) gebildet werden.

7. Verfahren nach einem der vorstehenden Ansprüche, bei dem die Ethylenfraktion als bis zu 20 mol.-% Ethan enthaltende Ethylenfraktion und/oder die Ethanfraktion als bis zu 20 mol.-% Ethylen enthaltende Ethanfraktion gebildet wird.

8. Anlage zur Herstellung von Vinylacetat, mit einem oder mehreren ersten Anlageneinheiten (10), die dafür eingerichtet ist oder sind, Ethan und Sauerstoff unter Erhalt eines Ethan, Ethylen, Wasser, Essigsäure und Kohlendioxid enthaltenden ersten Produktgemischs (11) einer oxidativen katalytischen Umsetzung zu unterwerfen, und mit einer oder mehreren zweiten Anlageeinheiten (20), die dafür eingerichtet ist oder sind, Ethylen und Essigsäure unter Erhalt eines Ethylen, Vinylacetat und Kohlendioxid enthaltenden gasförmigen zweiten Produktgemischs (21) einer katalytischen Vinylacetatsynthese zu unterwerfen, wobei die Anlage dafür eingerichtet ist, zumindest ein Teil des Ethylens und zumindest ein Teil der Essigsäure, die in der einen oder den mehreren Anlagenkomponenten (20) der katalytischen Umsetzung unterworfen werden, durch zumindest einen Teil des Ethylens und zumindest einen Teil der Essigsäure aus dem ersten Produktgemisch (11) zu bilden, **dadurch gekennzeichnet,**
- **dass** die Anlage dafür eingerichtet ist, unter Verwendung zumindest eines Teils des ersten Produktgemischs (11) ein Ethan, Ethylen und Kohlendioxid enthaltendes erstes Folgegemisch (12) zu bilden, wobei die Bildung des ersten Folgegemischs (12) eine zumindest teilweise Entfernung von Sauerstoff, Kohlenmonoxid und/oder Acetylen umfasst, und ferner umfasst, zumindest einen Teil des ersten Produktgemischs (11) unter Erhalt einer Wasser und Essigsäure enthaltenden Kondensatfraktion (41) und des ersten Folgegemischs (12) einer kondensativen Abscheidung (40) von Wasser und Essigsäure zu unterwerfen und wobei zumindest ein Teil der Essigsäure, die in dem einen oder den mehreren zweiten Verfahrensschritten (20) der katalytischen Vinylacetatsynthese unterworfen wird, durch zumindest einen Teil der Essigsäure aus der Kondensatfraktion (41) gebildet wird,
- **dass** die Anlage dafür eingerichtet ist, unter Verwendung zumindest eines Teils des zweiten Produktgemischs (21) ein Ethylen und Kohlendioxid enthaltendes zweites Folgegemisch (22) zu bilden, wobei die Bildung des zweiten Folgegemischs (22) umfasst, zumindest einen Teil des zweiten Produktgemischs (21) unter Erhalt einer Vinylacetat enthaltenden Vorproduktfraktion (61) und des zweiten Folgegemischs (22) einer Vinylacetatabtrennung (61) zu unterwerfen,
- **dass** die Anlage dafür eingerichtet ist, durch Zusammenführen von jeweils zumindest einem überwiegenden Teil des ersten Folgegemischs (12) und des zweiten Folgegemischs (22) ein Ethan, Ethylen und Kohlendioxid enthaltendes drittes Folgegemisch (31) zu bilden,
- **dass** die Anlage eine oder mehrere dritte Anlagenkomponenten (30) aufweist, die dafür eingerichtet ist oder sind, zumindest ein Teil des dritten Folgegemischs (31) einer Kohlendioxidentfernung zu unterwerfen, und die dafür eingerichtet ist oder sind, ein Ethan und Ethylen enthaltendes viertes Folgegemisch (32) und eine Kohlendioxidfraktion (33) zu bilden, wobei zumindest ein Teil des vierten Folgegemischs (32) einer Trennung (85, 90) unterworfen wird, in der eine Ethanfraktion und eine Ethylenfraktion gebildet werden,
- **dass** die Anlage dafür eingerichtet ist, zumindest einen Teil des Ethylens, das in dem einen oder den mehreren zweiten Verfahrensschritten (20) der katalytischen Vinylacetatsynthese unterworfen wird, durch zumindest ein Teil des Ethylens aus der Ethylenfraktion zu bilden, und/oder zumindest ein Teil des Ethans, das in dem einen oder den mehreren ersten Verfahrensschritten (20) der oxidativen katalytischen Dehydrierung unterworfen wird, durch zumindest einen Teil des Ethans aus der Ethanfraktion zu bilden, und
- **dass** die Anlage dafür eingerichtet ist, zumindest ein Teil des vierten Folgegemischs und/oder zumindest ein Teil der Ethylenfraktion einer Demethanisierung (85) zu unterwerfen.

## Claims

1. Process for producing vinyl acetate, in which in one or more first process steps (10), ethane and oxygen are subjected to oxidative catalytic dehydrogenation to obtain a first product mixture (11) containing ethane, ethylene, water, acetic acid and carbon dioxide, and in which in one or more second process steps (20), ethylene and acetic acid are subjected to catalytic vinyl acetate synthesis to obtain a gaseous second product mixture (21) containing ethylene, vinyl acetate and carbon dioxide, at least a portion of the ethylene and at least a portion of the acetic acid, which are subjected to the catalytic conversion in the one or more second process steps (20), being formed by at least a portion of the ethylene and at least a portion of the acetic acid from the first product mixture (11), **characterized in that**
- using at least a portion of the first product mixture (11), a first resultant mixture (12) containing ethane, ethylene and carbon dioxide is formed, the formation of the first resultant mixture (12) comprising an at least partial removal of oxygen, carbon monoxide and/or acetylene, and further comprising subjecting at least a portion of the first product mixture (11) to condensative separation (40) of water and acetic acid to obtain the first resultant mixture (12) and a condensate fraction (41) containing water and acetic acid, and at least a portion of the acetic acid, which is subjected to the catalytic vinyl acetate synthesis in the one or more second process steps (20), being formed by at least a portion of the acetic acid from the condensate fraction (41),
- **in that,** using at least a portion of the second product mixture (21), a second resultant mixture (22) containing ethylene and carbon dioxide is formed, the formation of the second resultant mixture (22) comprising subjecting at least a portion of the second product mixture (21) to vinyl acetate separation (60) to obtain the second resultant mixture (22) and a precursor fraction (61) containing vinyl acetate,
- **in that,** by combining at least a predominant portion of both the first resultant mixture (12) and the second resultant mixture (22), a third resultant mixture (31) containing ethane, ethylene and carbon dioxide is formed,
- **in that** at least a portion of the third resultant mixture (31) is subjected to one or more third process steps (30), the one or more third process steps (30) comprising carbon dioxide removal, a carbon dioxide fraction (33) and a fourth resultant mixture (32) containing ethane and ethylene being formed in the one or more third process steps (30), and at least a portion of the fourth resultant mixture (32) being subjected to a separation (85, 90) in which an ethane fraction and an ethylene fraction are formed,
- **in that** at least a portion of the ethylene, which is subjected to the catalytic vinyl acetate synthesis in the one or more second process steps (20), is formed by at least a portion of the ethylene from the ethylene fraction, and/or at least a portion of the ethane, which is subjected to the oxidative catalytic dehydrogenation in the one or more first process steps (20), is formed by at least a portion of the ethane from the ethane fraction, and
- **in that** at least a portion of the fourth resultant mixture and/or at least a portion of the ethylene fraction is subjected to demethanization (85).

2. Process according to claim 1, in which the carbon dioxide removal comprises regenerative adsorptive carbon dioxide removal, in which a scrubbing liquid is used which is loaded with carbon dioxide during the regenerative absorptive carbon dioxide removal, and from which the carbon dioxide is stripped to obtain the carbon dioxide fraction (33).

3. Process according to either of the preceding claims, in which at least a portion of the carbon dioxide fraction (33) is passed through the one or more second process steps (20) as a diluent.

4. Process according to any of the preceding claims, in which the formation of the third resultant mixture (31) comprises subjecting at least a portion of the first resultant mixture (12) and at least a portion of the second resultant mixture (22) to a common compression (45).

5. Process according to any of the preceding claims, in which a portion of the acetic acid from the condensate fraction (41) is used as an absorbent in the vinyl acetate separation (60).

6. Process according to any of the preceding claims, in which at least a portion of the precursor fraction (61) is subjected to vinyl acetate purification (70), in which a vinyl acetate fraction (71), a by-product fraction (72) and an acetic acid fraction (73) are formed.

7. Process according to any of the preceding claims, in which the ethylene fraction is formed as an ethylene fraction containing up to 20 mol.% ethane and/or the ethane fraction is formed as an ethane fraction containing up to 20 mol% ethylene.

8. Plant for producing vinyl acetate, comprising one or more first plant units (10) which is or are designed to subject ethane and oxygen to oxidative catalytic conversion to obtain a first product mixture (11) containing ethane, ethylene, water, acetic acid and carbon dioxide, and comprising one or more second plant units (20) which is or are designed to subject ethylene and acetic acid to catalytic vinyl acetate synthesis to obtain a gaseous second product mixture (21) containing ethylene, vinyl acetate and carbon dioxide, the plant being designed to form at least a portion of the ethylene and at least a portion of the acetic acid, which are subjected to the catalytic conversion in the one or more plant components (20), using at least a portion of the ethylene and at least a portion of the acetic acid from the first product mixture (11), **characterized in that**
- the plant is designed to form a first resultant mixture (12) containing ethane, ethylene and carbon dioxide using at least a portion of the first product mixture (11), the formation of the first resultant mixture (12) comprising an at least partial removal of oxygen, carbon monoxide and/or acetylene, and further comprising subjecting at least a portion of the first product mixture (11) to condensative separation (40) of water and acetic acid to obtain the first resultant mixture (12) and a condensate fraction (41) containing water and acetic acid, and at least a portion of the acetic acid, which is subjected to the catalytic vinyl acetate synthesis in the one or more second process steps (20), being formed by at least a portion of the acetic acid from the condensate fraction (41),
- **in that** the plant is designed to form a second resultant mixture (22) containing ethylene and carbon dioxide using at least a portion of the second product mixture (21), the formation of the second resultant mixture (22) comprising subjecting at least a portion of the second product mixture (21) to vinyl acetate separation (61) to obtain the second resultant mixture (22) and a precursor fraction (61) containing vinyl acetate,
- **in that** the plant is designed to form a third resultant mixture (31) containing ethane, ethylene and carbon dioxide by combining at least a predominant portion of the first resultant mixture (12) and of the second resultant mixture (22),
- **in that** the plant has one or more third plant components (30) which is or are designed to subject at least a portion of the third resultant mixture (31) to carbon dioxide removal, and which is or are designed to form a carbon dioxide fraction (33) and a fourth resultant mixture (32) containing ethane and ethylene, at least a portion of the fourth resultant mixture (32) being subjected to a separation (85, 90) in which an ethane fraction and an ethylene fraction are formed,
- **in that** the plant is designed to form at least a portion of the ethylene, which is subjected to the catalytic vinyl acetate synthesis in the one or more second process steps (20), using at least a portion of the ethylene from the ethylene fraction, and/or to form at least a portion of the ethane, which is subjected to the oxidative catalytic dehydrogenation in the one or more first process steps (20), using at least a portion of the ethane from the ethane fraction, and
- **in that** the plant is designed to subject at least a portion of the fourth resultant mixture and/or at least a portion of the ethylene fraction to demethanization (85).

## Revendications

1. Procédé de fabrication d'acétate de vinyle, dans lequel, dans une ou plusieurs premières étapes de procédé (10), de l'éthane et de l'oxygène sont soumis à une déshydrogénation catalytique oxydante pour obtenir un premier mélange de produits (11) contenant de l'éthane, éthylène, eau, acide acétique et dioxyde de carbone, et dans lequel, dans une ou plusieurs deuxièmes étapes de procédé (20), de l'éthylène et de l'acide acétique sont soumis à une synthèse catalytique d'acétate de vinyle pour obtenir un deuxième mélange de produits (21) gazeux contenant de l'éthylène, acétate de vinyle et dioxyde de carbone, dans lequel au moins une partie de l'éthylène et au moins une partie de l'acide acétique qui sont soumis à la réaction catalytique dans la ou les deuxièmes étapes de procédé (20) sont formés par au moins une partie de l'éthylène et au moins une partie de l'acide acétique provenant du premier mélange de produits (11), **caractérisé en ce**
- **que,** en utilisant au moins une partie du premier mélange de produits (11), un premier mélange subséquent (12) contenant de l'éthane, éthylène et dioxyde de carbone est formé, dans lequel la formation du premier mélange subséquent (12) comprend une élimination au moins partielle d'oxygène, monoxyde de carbone et/ou acétylène, et comprend en outre la soumission d'au moins une partie du premier mélange de produits (11) à une séparation par condensation (40) d'eau et d'acide acétique pour obtenir une fraction de condensat (41) contenant de l'eau et acide acétique et le premier mélange subséquent (12), et dans lequel au moins une partie de l'acide acétique qui est soumis à la synthèse catalytique d'acétate de vinyle dans la ou les deuxièmes étapes de procédé (20) est formée par au moins une partie de l'acide acétique provenant de la fraction de condensat (41),
- **que,** en utilisant au moins une partie du deuxième mélange de produits (21), un deuxième mélange subséquent (22) contenant de l'éthylène et dioxyde de carbone est formé, dans lequel la formation du deuxième mélange subséquent (22) comprend la soumission d'au moins une partie du deuxième mélange de produits (21) à une séparation d'acétate de vinyle (60) pour obtenir une fraction de précurseur (61) contenant de l'acétate de vinyle et le deuxième mélange subséquent (22),
- **qu'**un troisième mélange subséquent (31) contenant de l'éthane, éthylène et dioxyde de carbone est formé en réunissant respectivement au moins une partie prédominante du premier mélange subséquent (12) et du deuxième mélange subséquent (22),
- **qu'**au moins une partie du troisième mélange subséquent (31) est soumise à une ou plusieurs troisièmes étapes de procédé (30), dans lequel la ou les troisièmes étapes de procédé (30) comprennent une élimination de dioxyde de carbone, dans lequel, dans la ou les troisièmes étapes de procédé (30), un quatrième mélange subséquent (32) contenant de l'éthane et éthylène et une fraction de dioxyde de carbone (33) sont formés, et dans lequel au moins une partie du quatrième mélange subséquent (32) est soumise à une séparation (85, 90) dans laquelle une fraction d'éthane et une fraction d'éthylène sont formées,
- **qu'**au moins une partie de l'éthylène qui est soumis à la synthèse catalytique d'acétate de vinyle dans la ou les deuxièmes étapes de procédé (20) est formée par au moins une partie de l'éthylène provenant de la fraction d'éthylène, et/ou au moins une partie de l'éthane qui est soumis à la déshydrogénation catalytique oxydante dans la ou les premières étapes de procédé (20) est formée par au moins une partie de l'éthane provenant de la fraction d'éthane, et
- **qu'**au moins une partie du quatrième mélange subséquent et/ou au moins une partie de la fraction d'éthylène sont soumises à une déméthanisation (85).

2. Procédé selon la revendication 1, dans lequel l'élimination de dioxyde de carbone comprend une élimination de dioxyde de carbone par adsorption régénérative dans laquelle un liquide de lavage est utilisé, lequel est chargé en dioxyde de carbone lors de l'élimination de dioxyde de carbone par absorption régénérative, et à partir duquel le dioxyde de carbone est expulsé pour obtenir la fraction de dioxyde de carbone (33).

3. Procédé selon l'une des revendications précédentes, dans lequel au moins une partie de la fraction de dioxyde de carbone (33) est guidée à travers la ou les deuxièmes étapes de procédé (20) en tant que diluant.

4. Procédé selon l'une des revendications précédentes, dans lequel la formation du troisième mélange subséquent (31) comprend la soumission d'au moins une partie du premier mélange subséquent (12) et au moins une partie du deuxième mélange subséquent (22) à une compression commune (45).

5. Procédé selon l'une des revendications précédentes, dans lequel, dans la séparation d'acétate de vinyle (60), une partie de l'acide acétique provenant de la fraction de condensat (41) est utilisée en tant qu'absorbant.

6. Procédé selon l'une des revendications précédentes, dans lequel au moins une partie de la fraction de précurseur (61) est soumise à une purification d'acétate de vinyle (70) dans laquelle une fraction d'acétate de vinyle (71), une fraction de sous-produit (72) et une fraction d'acide acétique (73) sont formées.

7. Procédé selon l'une des revendications précédentes, dans lequel la fraction d'éthylène est formée sous forme d'une fraction d'éthylène contenant jusqu'à 20 % en moles d'éthane et/ou la fraction d'éthane est formée sous forme d'une fraction d'éthane contenant jusqu'à 20 % en moles d'éthylène.

8. Système de fabrication d'acétate de vinyle, comportant une ou plusieurs premières unités de système (10) qui sont configurées pour soumettre de l'éthane et de l'oxygène à une réaction catalytique oxydante pour obtenir un premier mélange de produits (11) contenant de l'éthane, éthylène, eau, acide acétique et dioxyde de carbone, et comportant une ou plusieurs deuxièmes unités de système (20) qui sont configurées pour soumettre de l'éthylène et de l'acide acétique à une synthèse catalytique d'acétate de vinyle pour obtenir un deuxième mélange de produits (21) gazeux contenant de l'éthylène, acétate de vinyle et dioxyde de carbone, dans lequel le système est configuré pour former au moins une partie de l'éthylène et au moins une partie de l'acide acétique qui sont soumis à la réaction catalytique dans le ou les composants de système (20) par au moins une partie de l'éthylène et au moins une partie de l'acide acétique provenant du premier mélange de produits (11), **caractérisé en ce**
- **que,** le système est configuré pour former un premier mélange subséquent (12) contenant de l'éthane, éthylène et dioxyde de carbone en utilisant au moins une partie du premier mélange de produits (11), dans lequel la formation du premier mélange subséquent (12) comprend une élimination au moins partielle d'oxygène, monoxyde de carbone et/ou acétylène, et comprend en outre la soumission d'au moins une partie du premier mélange de produits (11) à une séparation par condensation (40) d'eau et d'acide acétique pour obtenir une fraction de condensat (41) contenant de l'eau et acide acétique et le premier mélange subséquent (12), et dans lequel au moins une partie de l'acide acétique qui est soumis à la synthèse catalytique d'acétate de vinyle dans une ou plusieurs deuxièmes étapes de procédé (20) est formée par au moins une partie de l'acide acétique provenant de la fraction de condensat (41),
- **que,** le système est configuré pour former un deuxième mélange subséquent (22) contenant de l'éthylène et dioxyde de carbone en utilisant au moins une partie du deuxième mélange de produits (21), dans lequel la formation du deuxième mélange subséquent (22) comprend la soumission d'au moins une partie du deuxième mélange de produits (21) à une séparation d'acétate de vinyle (61) pour obtenir une fraction de précurseur (61) contenant de l'acétate de vinyle et le deuxième mélange subséquent (22),
- **que** le système est configuré pour former un troisième mélange subséquent (31) contenant de l'éthane, éthylène et dioxyde de carbone en réunissant respectivement au moins une partie prédominante du premier mélange subséquent (12) et du deuxième mélange subséquent (22),
- **que** le système présente un ou plusieurs troisièmes composants de système (30) qui sont configurés pour soumettre au moins une partie du troisième mélange subséquent (31) à une élimination de dioxyde de carbone et qui sont configurés pour former un quatrième mélange subséquent (32) contenant de l'éthane et éthylène et une fraction de dioxyde de carbone (33), dans lequel au moins une partie du quatrième mélange subséquent (32) est soumise à une séparation (85, 90) dans laquelle une fraction d'éthane et une fraction d'éthylène sont formées,
- **que** le système est configuré pour former au moins une partie de l'éthylène qui est soumis à la synthèse catalytique d'acétate de vinyle dans la ou les deuxièmes étapes de procédé (20) par au moins une partie de l'éthylène provenant de la fraction d'éthylène, et/ou pour former par au moins une partie de l'éthane provenant de la fraction d'éthane au moins une partie de l'éthane qui est soumis à la déshydrogénation catalytique oxydante dans la ou les premières étapes de procédé (20), et
- **que** le système est configuré pour soumettre au moins une partie du quatrième mélange subséquent et/ou au moins une partie de la fraction d'éthylène à une déméthanisation (85).
